(19) Europäisches Patentamt
European Patent Office
Office européen des brevets

(11) **EP 4 433 572 B1**

(12) **EUROPEAN PATENT SPECIFICATION**

(45) Date of publication and mention
of the grant of the patent:
**24.12.2025 Bulletin 2025/52**

(21) Application number: **22818671.4**

(22) Date of filing: **17.11.2022**

(51) International Patent Classification (IPC):
***C12M 3/06*** *(2006.01)*

(52) Cooperative Patent Classification (CPC):
**C12M 27/16; B01L 3/502761; C12M 23/16;**
B01L 2300/0816; B01L 2300/0877;
B01L 2300/0883; B01L 2400/0409;
B01L 2400/0457; B01L 2400/0487

(86) International application number:
**PCT/EP2022/082301**

(87) International publication number:
**WO 2023/089043 (25.05.2023 Gazette 2023/21)**

(54) **MICROFLUIDIC DEVICE**

MIKROFLUIDISCHE VORRICHTUNG

DISPOSITIF MICROFLUIDIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **17.11.2021 EP 21208839**

(43) Date of publication of application:
**25.09.2024 Bulletin 2024/39**

(73) Proprietor: **Insphero AG
8952 Schlieren (CH)**

(72) Inventors:
• **FREY, Olivier
4104 Oberwil (CH)**
• **LOHASZ, Christian
79100 Freiburg im Breisgau (DE)**
• **MODENA, Mario Matteo
79102 Freiburg im Breisgau (DE)**
• **MISUN, Patrick Mark
4411 Seltisberg (CH)**
• **RENGGLI, Kasper
4056 Basel (CH)**

(74) Representative: **Michalski Hüttermann & Partner
Patentanwälte mbB
Kaistraße 16A
40221 Düsseldorf (DE)**

(56) References cited:
WO-A2-2006/037033      CA-A1- 3 142 903
US-A1- 2019 009 274

## Description

## Field of the invention

The present invention relates to the field of microfluidic devices for use in cell cultures and tissue cultures, or testing thereof.

## Introduction

3D tissue cultures are more and more used in the testing or screening of compounds for their toxicity, immunogenicity or therapeutic effect. In one specific embodiment, an array of different or similar 3D tissue cultures connected to one another is exposed to the same test compound, to (i) investigate the different effects of the compound on the different tissues and (ii) be able to consider potential communication or cross-talk between the different tissues in response to such exposure.

These devices are sometimes called "organ-on-a-chip", or even "organism-on-a-chip" or "body-on-a-chip". More recently they are summarized as "microphysiological systems" (MPS). Microphysiological systems combine microfluidic technology and cell/tissue culturing and are capable of emulating human (or any other animal species') biology *in vitro* at the smallest biologically acceptable scale, defined by purpose.

There are many devices and methods published according to which, for example, microtissue spheroids can be formed inside a chip and cultured there under flow over time. See Ruppen et al. (2015), Occhetta et al. (2015), Kwapiszewska et al. (2014), Jin et al. (2011), Hsiao et al. (2009), Torisawa et al. (2007) or Wu et al (2008)

Patent application WO 2006/037033 discloses microfluidic devices for controlled cell growth; CA 3142903 discloses centrifugal microfluidic chips and kits for controlled gas supply to the chips; US 2019/009274 discloses microfluidic devices with flow by gravity. None of the patent applications above disclose or suggest specific angles between flow directions and gravity or centrifugal forces as technical features.

Only a few exist, however, in which microtissue spheroids are formed externally by using dedicated platforms and only then transferred and loaded into a microfluidic culturing chip. For this reason, microtissue spheroids, organoids, precision cut tissue slices, 3D tissue spheroids, embryoid bodies and islets will be referred to as "microtissues" hereinafter. All disclosures and conclusions made herein with regard to microtissue spheroids do apply to organoids, precision cut tissue slices, 3D tissue spheroids, embryoid bodies and islets as specimen.

Microfluidic devices are systems which typically feature small liquid volumes and small diameters of liquid conduits. They are oftentimes characterized by laminar liquid flows within the conduits.

Generally, liquid flows in elongated tubing can be turbulent or laminar. The Reynolds number (Re) helps predict such flow patterns in different fluid flow situations. For low Reynolds numbers, flow is dominated by shearing forces and is laminar, while at high Reynolds numbers flow is dominated by inertial forces and is turbulent. The Reynolds number is defined as:

$$\mathrm{Re} = \frac{\rho v l}{\mu} = \frac{v l}{\nu}$$

where

- $\rho$ is the density of the liquid (SI units: $kg/m^3$)
- $v$ is the flow speed (m/s)
- $l$ is a characteristic linear dimension (m), e.g. the diameter of the conduit
- $\mu$ is the dynamic viscosity of the fluid ($Pa \cdot s$ or $N \cdot s/m^2$ or $kg/(m \cdot s)$) and
- $\nu$ is the kinematic viscosity of the fluid ($m^2/s$).

Due to the small diameters of the conduits, microfluidic devices often have a Reynolds number of < 2300, which marks the border below which laminar flow is predominant.

Contrary to a system in which turbulent flows are predominant, in microfluidic devices, non-soluble components of the liquid have hence the tendency to settle, or sediment, caused by gravitational (or centrifugal) forces. This applies *inter alia,* to cells that are comprised in a liquid that flows through a microfluidic device.

Microfluidic devices are oftentimes used in tissue or cell biology, where microtissues are exposed to media comprising agents like drugs or toxins. As long as these agents are soluble in the liquid, sedimentation thereof is not a major problem even in laminar flow systems.

Some applications do however require the investigation of synergism between free-floating cells or non-soluble particles, e.g. nanomaterials, and such microtissues.

In immunoncology, the effect of cells of the immune system on cancerous (solid) tissues is investigated, oftentimes in combination with a drug candidate, like e.g. an immune checkpoint inhibitor, a bispecific T cell engaging antibody or an antibody having ADCC (antibody dependent cellular cytotoxicity) activity. Another example are circulating tumor cells (CTC), which may or may not interact with microtissues, e.g., in their role as organ models.

Also in other applications, where for example the effect of a drug on overshooting immune response is investigated, cells of the immune system are brought in contact with a target microtissue.

In other application, cells are investigated under flow conditions, with or without interaction with drug candidates.

In all these applications, sedimentation of non-soluble cells can indeed become a problem, because the risk exists that the respective cells do not reach the microtissues that are subject of the investigation and/or their settling and leaving of the flow has adverse effects of the cell (e.g. cell death, cell accumulation).

This also applies to non-soluble particles that are comprised in the fluid, for example to study interactions between the microtissue and such particles.

Such particles are for example

- liposomes comprising e.g. mRNA or other genetic material,
- viral vectors,
- colloids (silver, silicates),
- nano- and microplastic particles,
- ZnO, SiO2, CeO2, BaSO4, TiO2, and/or
- beads coated with active agents, like e.g. antibodies.

Hence, the entire discussion provided herein for cells in the culture liquid also apply to non-soluble particles prone to gravitational sedimentation.

## Summary of the invention

It is one object of the present invention to provide a microfluidic device that is suitable for using cells and/or particles which circulate through the conduits of the system.

It is one object of the present invention to provide a microfluidic device which allows to study the interaction between microtissues, cells and/or particles, optionally in the presence of an agent that affects such interaction or is being affected by such interaction.

It is another object of the present invention to provide a microfluidic device which reduces sedimentation of cells and/or particles which circulate through the conduits of the system.

These and further objects are achieved by the subject matter of the independent claims, while the dependent claims as well as the specification disclose further preferred embodiments.

## Embodiments of the invention

Before the invention is described in detail, it is to be understood that this invention is not limited to the particular component parts or structural features of the devices or compositions described or process steps of the methods described as such devices and methods may vary. It is also to be understood that the terminology used herein is for purposes of describing particular embodiments only, and is not intended to be limiting. The mere fact that certain measures are recited in mutually different dependent claims does not indicate that a combination of these measures cannot be used to advantage. Any reference signs in the claims should not be construed as limiting the scope. It must be noted that, as used in the specification and the appended claims, the singular forms "a," "an" and "the" include singular and/or plural referents unless the context clearly dictates otherwise. Further, in the claims, the word "comprising" does not exclude other elements or steps.

It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, for prior art documents that disclose standard or routine methods. In that case, the incorporation by reference has mainly the purpose to provide sufficient enabling disclosure, and avoid lengthy repetitions.

According to one aspect of the invention, a microfluidic device suitable for accommodating an aqueous liquid is provided, the device having essentially a planar shape. The device comprises at least one conduit fluidically connecting at least two structures selected from compartments, reservoirs and/or ports.

Said conduit comprises at least one bend ("meander") describing an angle of between $\geq 85°$ and $\leq 275°$ around an axis orthogonal to the planar shape, which planar shape is arranged in an angle of between +45° and -45° relative to the direction of

    a) the force of gravity (G), or
    b) a centrifugal force (Fc) applied to the microfluidic device

In different embodiments, the bend describes an angle of 85°, 86°, 87°, 88°, 89°, 90°, 91°, 92°, 93°, 94°, 95°, 96°, 97°, 98°, 99°, 100°, 101°, 102°, 103°, 104°, 105°, 106°, 107°, 108°, 109°, 110°, 111°, 112°, 113°, 114°, 115°, 116°, 117°, 118°, 119°, 120°, 121°, 122°, 123°, 124°, 125°, 126°, 127°, 128°, 129°, 130°, 131°, 132°, 133°, 134°, 135°, 136°, 137°, 138°, 139°, 140°, 141°, 142°, 143°, 144°, 145°, 146°, 147°, 148°, 149°, 150°, 151°, 152°, 153°, 154°, 155°, 156°, 157°, 158°, 159°, 160°, 161°, 162°, 163°, 164°, 165°, 166°, 167°, 168°, 169°, 170°, 171°, 172°, 173°, 174°, 175°, 176°, 177°, 178°, 179°, 180°, 181°, 182°, 183°, 184°, 185°, 186°, 187°, 188°, 189°, 190°, 191°, 192°, 193°, 194°, 195°, 196°, 197°, 198°, 199°, 200°, 201°, 202°, 203°, 204°, 205°, 206°, 207°, 208°, 209°, 210°, 211°, 212°, 213°, 214°, 215°, 216°, 217°, 218°, 219°, 220°, 221°, 222°, 223°, 224°, 225°, 226°, 227°, 228°, 229°, 230°, 231°, 232°, 233°, 234°, 235°, 236°, 237°, 238°, 239°, 240°, 241°, 242°, 243°, 244°, 245°, 246°, 247°, 248°, 249°, 250°, 251°, 252°, 253°, 254°, 255°, 256°, 257°, 258°, 259°, 260°, 261°, 262°, 263°, 264°, 265°, 266°, 267°, 268°, 269°, 270°, 271°, 272°, 273°, 274° and/or 275°.

A preferred subrange is between ≥ 85° and ≤ 185°, between ≥ 130° and ≤ 185° and/or between ≥ 85° and ≤ 140°.

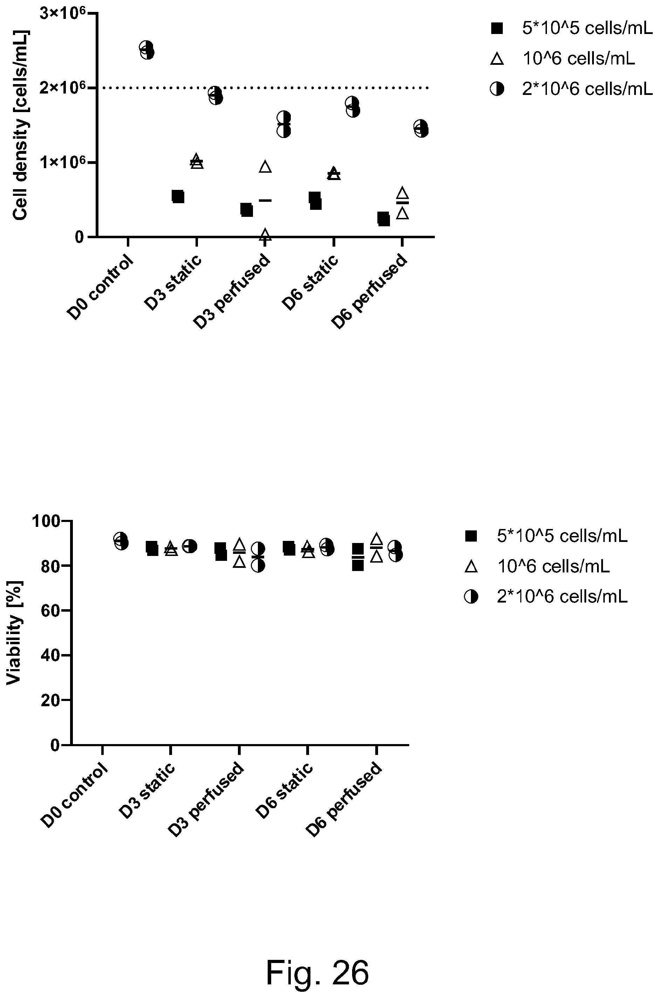

As used herein, the term at least one bend describing an angle of between ≥ 85° and ≤ 275° around an axis "orthogonal to the planar shape" is to be understood in a broad fashion, and should also include embodiments where the conduit is not in parallel to the surface of the device, but inclined relative thereto, e.g., by forming a helical conduits structure. See Fig. 26 for a better explanation of this embodiment.

Such microfluidic device is a three-dimensional body that is defined by the three axis, X, Y, and Z. The planar shape is defined by X and Z. Z is the axis that is arranged in an angle of between +45° and -45° relative to the force of gravity, or the centrifugal force applied to the microfluidic device. The angle that defines the bend of the conduit is orthogonal to X and Z, hence around the Y axis.

In this regard, the term "planar" shape implies that the microfluidic device can also be called a chip, plate or slide.

In other embodiments, the planar shape is arranged in an angle of between +40° and -40°, +35° and -35°, +30° and -30°, +25° and -25°, +20° and -20°, +15° and -15°, +10° and -10°, or +5° and -5°.

According to one embodiment, the device further comprises at least one compartment arranged within the conduit, or between two sections thereof, said compartment being capable of accommodating at least one microtissue ("microtissue compartment").

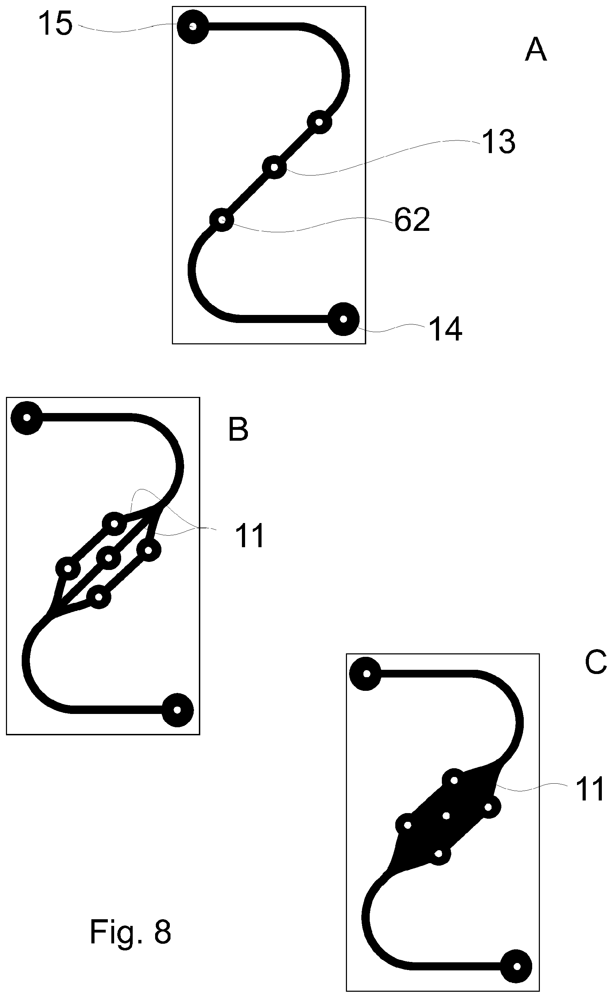

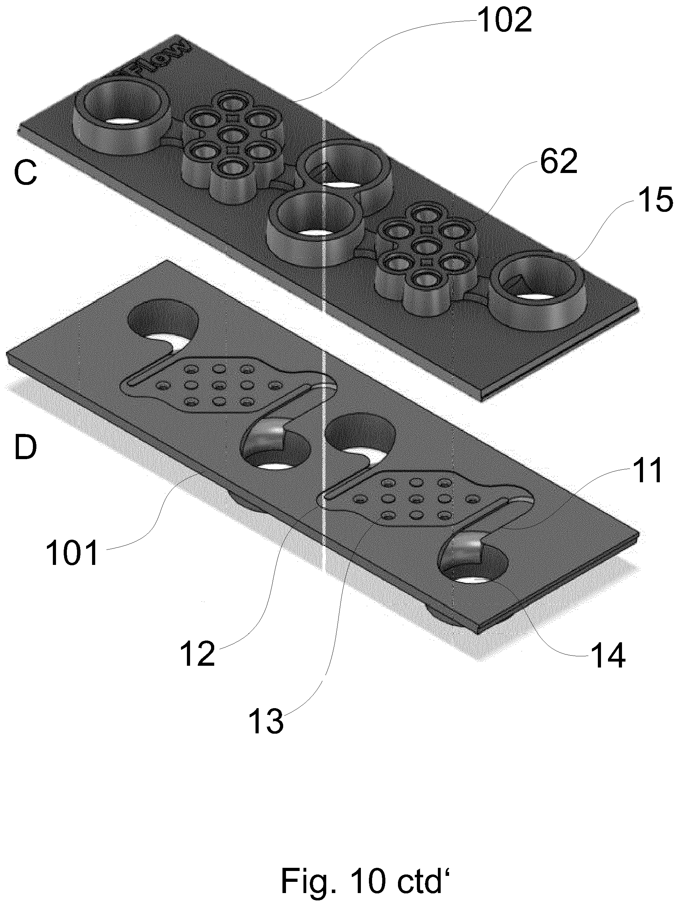

As used herein, the term "arranged within the conduit" shall refer to an embodiment in which the diameter of the conduit widens up and accommodates one or more compartments. Such embodiment is for example shown in Fig 8C or Fig. 10C and 10D.

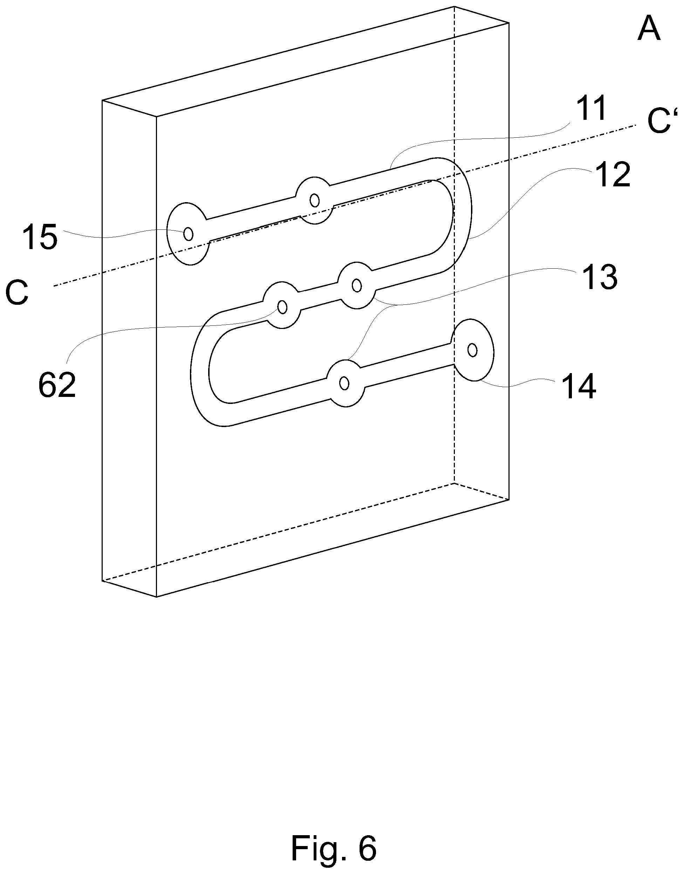

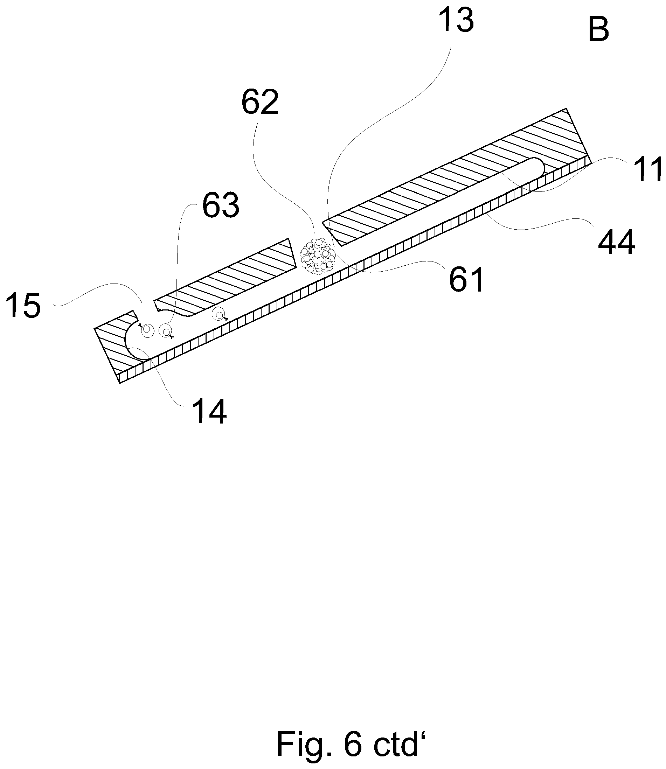

As used herein, the term "arranged between two sections of a conduit" shall refer to an embodiment in which at least one compartment is arranged between two sections of the conduit which do not widen up. Such embodiment is for example shown in Fig. 6, Fig 8A and 8B.

According to one embodiment, the conduit furcates into two or more conduits which are arranged in parallel, in which each of the two or more conduits comprises at least one compartment arranged between two sections thereof.

According to one embodiment at least one compartment has a circular cross section, and has a compartment port with a circular cross section arranged concentrically to the compartment, and wherein the diameter of the compartment is larger than the diameter of the compartment port.

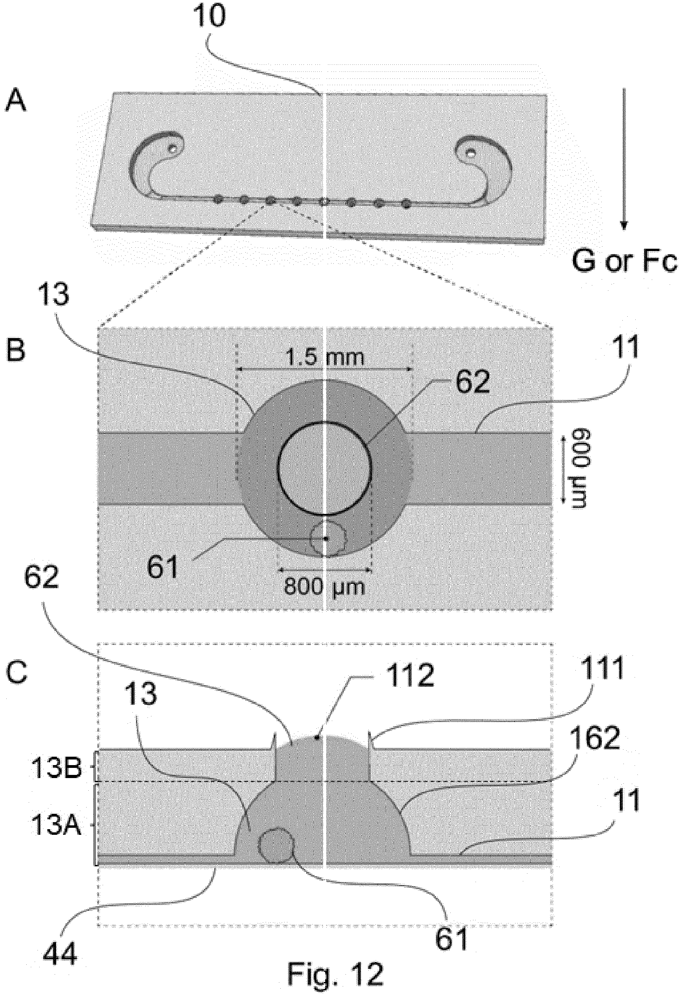

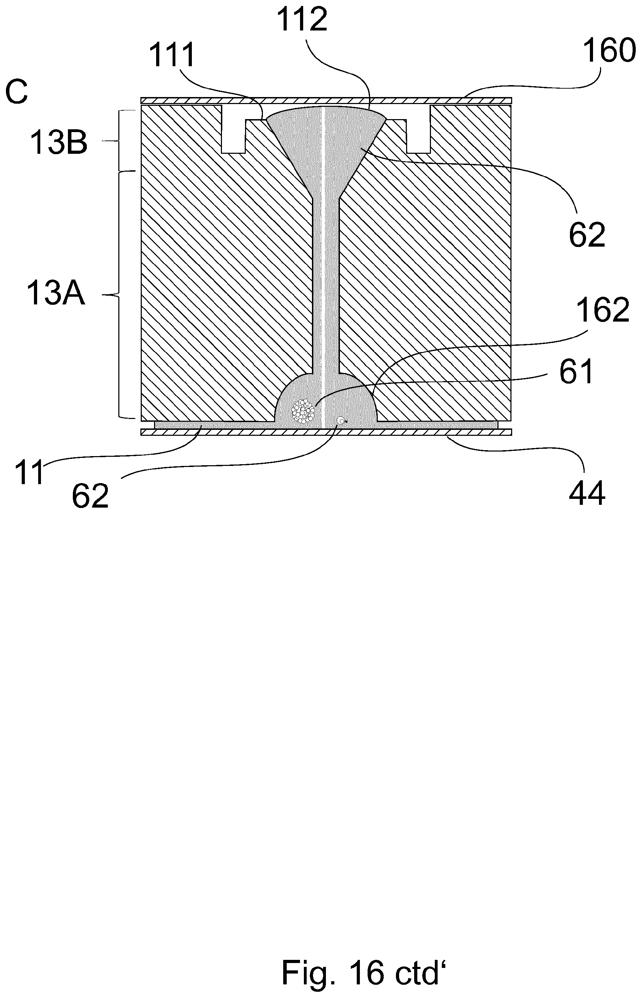

In such embodiment, the compartment may have a lower section adjacent to the conduit and an upper section, which is adjacent to a compartment port, as e.g. shown in Fig. 12C or Fig. 16C. In such embodiment, the compartment may have, in its lower section, a dome structure, to reduce the risk that a microtissue comprised in the compartment is washed out during operation of the microfluidic device. Tests have shown that, relative to a lower section that has a conical or funnel shaped tapering towards the compartment port the dome structure significantly reduces such risk.

Such dome structure may have a spheroidal or concave shape, compared to a simple conical or funnel shaped tapering.

It is important to mention that the said embodiment, with a compartment to accommodate a microtissue with a circular cross section, a lower section and upper section, a compartment port with a circular cross section arranged concentrically to the compartment, and a dome structure, shall also be deemed disclosed in isolated fashion, i.e., independent of the other elements of the microfluidic device, because such structure can be used advantageously also in other devices for storing or culturing microtissues, to reduce the risk that a microtissue comprised in the compartment is washed out during operation of the device.

In several embodiments the compartment has a spheroidal, ellipsoidal, prismatic, cubic, or cylindric shape.

In one embodiment, the compartment has a lower section which is adjacent to the two sections of a conduit and an upper section, which is adjacent to a compartment port, as e.g. shown in Fig. 16C.

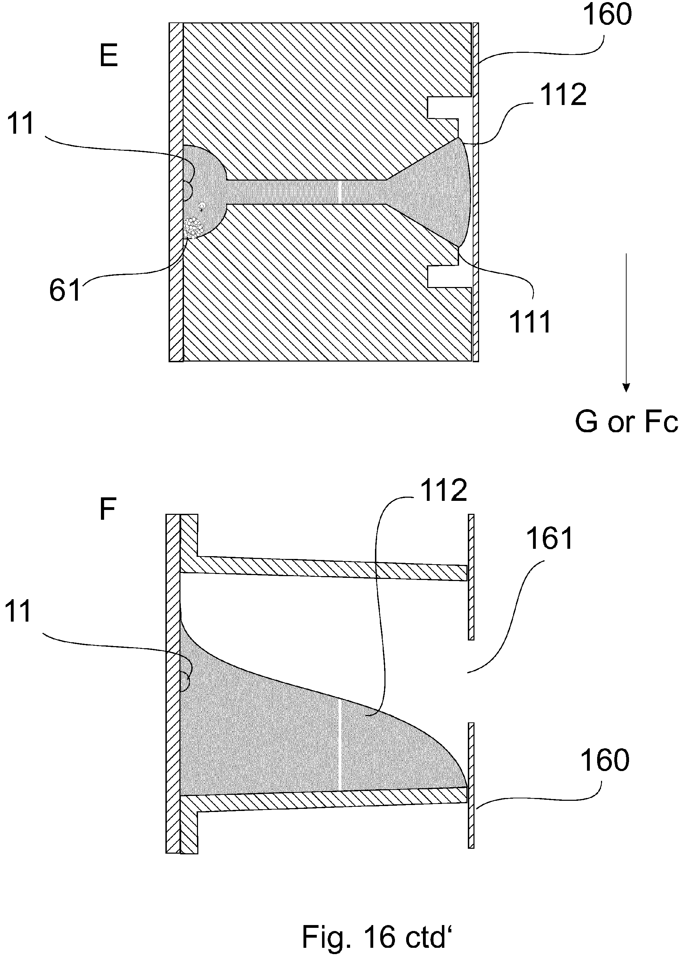

In one embodiment, said compartment has, in its lower section, a dome structure, as e.g. shown in Fig. 16 C and 16E, to reduce the risk that a microtissue comprised in the compartment is washed out during operation of the microfluidic device.

The diameter of the compartment may be chosen relative to the type of tissue or microtissue to be used.

In another embodiment, the compartment comprises an ultra-low attachment surface coating suitable for long-term maintenance of a microtissue, like e.g. used in ULA plates (Akura™ 96 spheroid microplates, In-Sphero AG)

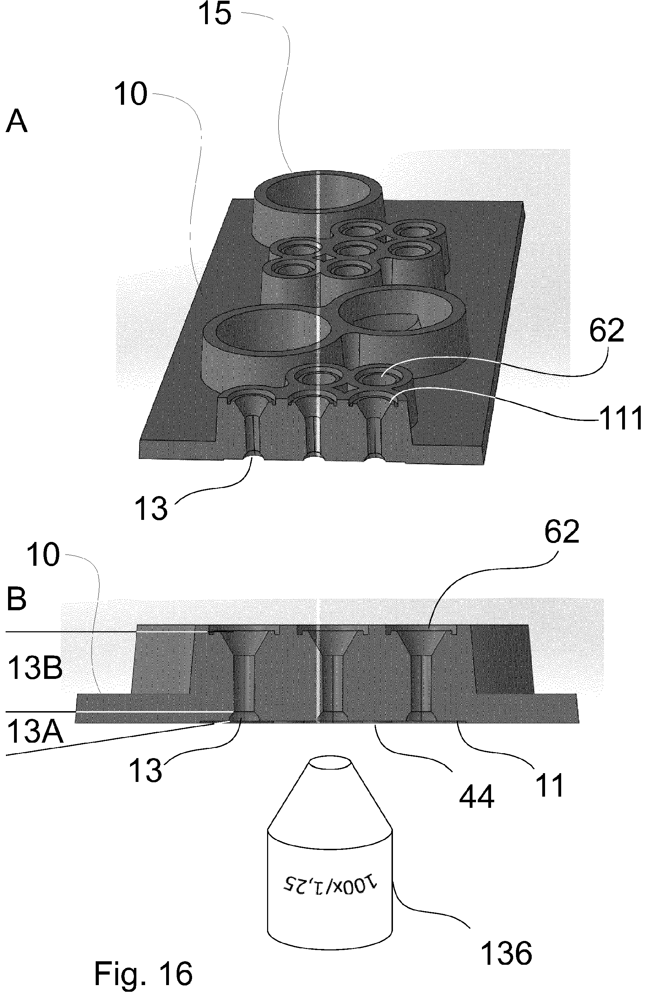

In a further embodiment the compartment comprises an optical detection area suitable for high-content imaging. Such optical detection area is in one embodiment formed by the compartment for accommodating at least one microtissue, and a transparent bottom layer, which may be formed, for example, by a transparent liner as described elsewhere herein. The microfluidic device may be placed on the stage of an inverted microscope, as e.g. shown in Fig. 16B.

In other embodiments, the microfluidic device comprises up to 1, 2, 3, 4, 5, 6, 7, 8, 9, 10, 12 24, 32, 48, 64, 96, 128, 192, 256 or 384 microtissue compartments.

The microtissue compartments can be arranged in sequence along the straight and/or bended part of the conduit (e.g. Fig 6 or Fig 8A), or arranged in parallel (e.g. Fig 8B, 8C or Fig 10).

The distance between the compartments can range between $\geq 0.5$ and $\leq 20$ mm, between $\geq 1$ and $\leq 10$ mm, between $\geq 2$ and $\leq 8$ mm, between $\geq 3$ and $\leq 5$, and in one preferred embodiment can be 9.0 mm and in another preferred embodiment can be 4.5 mm.

In one embodiment, the conduit comprises a series of bends ("meanders") with angles selected from the above list. In several embodiments, the conduit comprises 2 bends, 3 bends, 4 bends, 5 bends or 6 bends.

In one embodiment, said liquid is a cell culture medium. In one embodiment, said cell culture medium comprises cells and/or particles. In one embodiment, said cells are immune cells or circulating tumor cells (CTC), or a mixture thereof.

In several embodiments, the microfluidic device comprises a polymeric material, such as e.g. cyclo-olefin polymer, cyclic olefin copolymer, polycarbonates, polydimethylsiloxane, polyethylene, polyethylenefluoroethylene, polymethylmethacrylate, polypropylene, polysiloxanes, polystyrene, polyurethanes, polyvinyl chloride, or a combination of two or more of the foregoing.

According to one embodiment, at least one conduit has a polygonal, circular, ellipsoid, semicircular, or semiellipsoid cross section with at least one cross section dimension smaller than 250 $\mu$m.

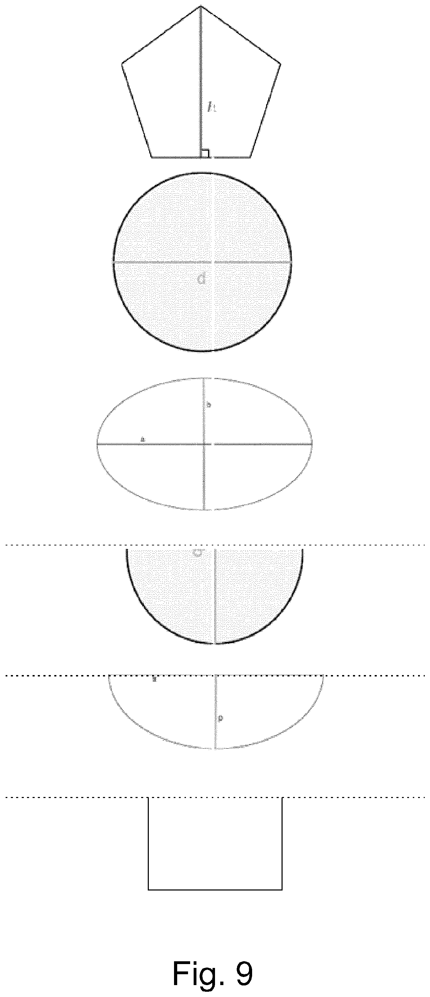

As used herein, the term "cross section dimension", related to any one of height (h) (in a conduit having a polygonal cross section), diameter (d) (in a conduit having a circular or semicircular cross section) or axis (b) (in a conduit having an ellipsoid or semiellipsoid cross section). See, in this regard. Fig. 9 and description.

The cross section dimension is selected in such way that microtissues of typical sizes cannot pass the conduit, so remain in the compartments (see Fig. 6B, Fig. 12C, Fig. 13 or Fig. 16C). In one embodiment, the cross section is smaller than 150 $\mu$m, which is for example particularly useful for islet microtissues. In one embodiment, the cross section is smaller than 220 $\mu$m, which is for example particularly useful for microtissues comprising hepatocytes. The compartment is confined by means of a standing drop (of cell culture medium).

According to one embodiment, the device further comprises, or is connected to or mountable on, a means to generate a flow of aqueous liquid within the conduit.

In one embodiment, the means to generate a flow of liquid is a tilting device or a tiltable device. Such tilting device can preferably perform a tilting movement, preferably around the X axis. In one embodiment, the means to generate a flow of liquid is a rotating device. In one embodiment, the direction of rotation is preferably around the Y axis. In one embodiment, the means to generate a flow of liquid is a pump. In one embodiment, such pump comprises a syringe pump. Two or three of these approaches can be combined. In one embodiment, a back and forth flow ("oscillating flow") is generated.

According to one embodiment, the reservoir is suitable to accommodate a liquid optionally comprising cells and/or particles. Such aqueous liquid is preferably a culture liquid, preferably a cell culture liquid or a microtissue culture liquid.

According to one embodiment, at least one port is suitable to be connected, or is connected, to an external tube or hose, which tube or hose is optionally connected to a pump.

As discussed herein, a port can be a conduit port (for connecting e.g. a pump), a compartment port (for e.g. loading microtissues), or a reservoir port (for e.g. taking out cells and/or media, or for adding an agent etc.).

According to one embodiment, the conduit has two ends each of which fluidically connects to a reservoir.

According to one embodiment, at least one reservoir comprises a reservoir port. In one embodiment, the reservoir has a circular or ellipsoid cross section. In one embodiment, the reservoir port is arranged coaxially to the reservoir.

According to one embodiment, the device comprises a base plate with a surface comprising at least one elongated groove, wherein on at least part of the respective surface of the base plate a liner is arranged so as to seal the elongated groove, and hence form the conduit.

In several embodiments, the base plate comprising the at least one elongated groove is fabricable by 3D printing, milling or molding.

In several embodiments, the elongated groove has a polygonal, semicircular, or semiellipsoid cross section. When sealed, at least one conduit has a polygonal, circular, ellipsoid, semicircular, or semiellipsoid cross section with at least one cross section dimension that is smaller than that of the microtissue, so as to avoid that the microtissue is carried out of the compartment by means of the aqueous liquid.

In one embodiment, the compartment comprises a port (compartment port), through which the microtissue can be loaded into the compartment.

In several embodiments, the base plate comprises a material selected from the group consisting of polystyrene, PMMA, COC, COP, PTFE, and aluminum, or a material selected from the group consisting of polydimethylsiloxane (PDMS) rubber, vinyl methyl siloxane, phenyl vinyl methyl siloxane, fluorosilicone rubber, and/or nitrile rubber and natural rubber.

In several embodiments, the liner comprises a material selected from the group consisting of polystyrene, PMMA, COC, COP, PTFE, and aluminum, or a material selected from the group consisting of polydimethylsiloxane (PDMS) rubber, vinyl methyl siloxane, phenyl vinyl methyl siloxane, fluorosilicone rubber, and/or nitrile rubber and natural rubber.

In another embodiment, the microfluidic device further comprises a lid which covers, fully or partially, at least one port selected out of the group of compartment ports and reservoir ports.

According to one embodiment, the lid comprises a foil or mat. In several embodiments, the foil or mat comprises a material selected from the group consisting of polystyrene, PMMA, COC, COP, PTFE, and aluminum, or a material selected from the group consisting of polydimethylsiloxane (PDMS) rubber, vinyl methyl siloxane, phenyl vinyl methyl siloxane, fluorosilicone rubber, and/or nitrile rubber and natural rubber.

According to another aspect of the invention, an assembly or array comprising a plurality of microfluidic devices according to the above description is provided. Such array can be provided as a frame which is capable to accommodate several microfluidic devices.

The array or frame can comprise, or accommodate, respectively, for example 2, 4, 6, 8, 10, 12, 14, 16, 18, 20 or even more microfluidic devices according to the above description.

According to one embodiment, the array comprises, or is mountable on or connectable to, at least one tilting unit, tiltable unit, rotating unit, and/or a pump.

According to another aspect of the invention, a method of culturing at least one microtissue and/or at least one cell in a microfluidic device according to the above description is provided, which method comprises,

• loading at least one microtissue and/or at least one cell into the microfluidic device, and, simultaneously, before or subsequently, introducing an aqueous liquid into the microfluidic device and

• establishing a liquid flow through the microfluidic device.

In this method, due to the bend provided in at least one conduit, the sedimentation of non-soluble components in the aqueous liquid is avoided.

According to one embodiment, the liquid flow is driven or supported by at least one of:

• a pump, and/or
• tilting, turning, pivoting or rotating the device.

The tilting, turning, rotating, or pivoting induces flow through gravity, in particular in case when there are two or more reservoirs. By turning, rotating, or pivoting, the latter constantly change their height relative to one another causing a changing hydrostatic pressure between the reservoirs and thus the liquid to flow.

In one embodiment, the direction of liquid flow is changed repeatedly. In such way a back and forth flow ("oscillating flow") is generated.

In one embodiment, after the microfluidic device has been tilted or rotated, a pause or break is introduced before the tilting or rotation is resumed in the opposite direction. Such pause or break can have a length of between ≥ 0,5 sec and ≤ 3600 sec. In preferred embodiments, the pause or break has a length of between ≥ 10 sec and ≤ 240 sec.

According to one embodiment, the aqueous liquid comprises cells and/or particles.

Such aqueous liquid is preferably a culture liquid, preferably a cell culture liquid or a microtissue culture liquid. Cells are non-soluble and have the tendency, in a laminar flow system, like a microfluidic conduit, to sediment on the inner surface, of said conduit, facing the direction of gravity or centrifugal force. As discussed above, the same applies for particles.

In one embodiment, the cells are immune cells. In one embodiment, the cells are cytotoxic T cells (CD8+). In one embodiment, the cells are T helper cells (CD4+). In one embodiment, the cells are NK cells (e.g., CD16+). In one embodiment, the cells are peripheral blood mononuclear cells (PBMCs). In one embodiment, the cells are granulocytes (basophils, eosinophils, neutrophils, and mast cells). In one embodiment, the cells are agranulocytes (lymphocytes, macrophages, monocytes, and natural killer (NK) cells). In one embodiment, the cells are stem cell-derived immune cells or immune cell lines.

In one embodiment, the cells express an immune checkpoint, e.g. PD-1, CTLA-4, Lag-3, In one embodiment, the cells express CD3, or a subdomain thereof, like e.g. CD3$\alpha$, CD3$\beta$, CD3$\epsilon$, CD3$\gamma$ or CD3$\delta$. In such embodiment, the agent that is investigated is for example a bispecific antibody comprising a CD3 binding domain, like e.g. a bispecific T cell engager (biTE).

In one embodiment, the cells express one or more Fc$\gamma$ receptors, like e.g. CD16 or Fc$\gamma$RIII. In such embodiment, the agent that is investigated is for example an ADCC eliciting antibody comprising an Fc domain.

In another embodiment, the cells are circulating tumor cells (CTC).

**Definitions**

As used herein, the term at least one bend describing an angle of between ≥ 85° and ≤ 275° around an axis "orthogonal to the planar shape" is to be understood in a broad fashion, and should also include embodiments where the conduit is not in parallel to the surface of the device, but inclined relative thereto, e.g., by forming a helical conduits structure. See Fig. 26 for a better explanation of this embodiment.

The term "planar" shape implies that the microfluidic device can also be called a chip, plate or slide.

The term "aqueous liquid", as used herein, relates to a liquid the major part of which consists of water. Such liquid may furthermore comprise organic or anorganic components.

The term "particles", as used herein, is meant to encompass non-soluble, non-degradable, or degradable particles of matter such as "microparticles" (1-1000 $\mu$m), "fine particles" (100-2500 nm), and "coarse particles" (2500-10000 nm). Such particles are selected, for example, from

- liposomes comprising e.g. mRNA or other genetic material,
- viral vectors,
- colloids (silver, silicates),
- nano- and microplastic particles,
- ZnO, SiO2, CeO2, BaSO4, TiO2, and/or
- beads coated with active agents, like e.g. antibodies.

The term "microtissue", as used herein, is meant to encompass 3D cell culture models. including organoids, 3D tissue spheroids, embryoid bodies, islets, precision cut tissue slides, and the like. The microtissues can be of natural origin, or can be engineered or passively or actively reassembled from isolated cells.

The microtissues may comprise primary cells obtained from organs and reassembled with suitable methods, as e.g. described in Messner et al (2013), to form such microtissues. The microtissues may comprise or consist of stem cells, immortalized cells or cell lines, or a mixture thereof. The microtissues may also comprise cancerous cells obtained from tumors in similar fashion, may comprise or consist of tumor microbiopsies, or may be derived from tumor biopsies. In one embodiment, the microtissue may comprise cancerous cells and healthy cells.

In other embodiments the microtissue cells are mammalian cells, preferably human cells, cynomolgus monkey cells, porcine cells, canine cells, rat cells or mouse cells. In other embodiments, the microtissues comprise hepatocytes, Kupffer and/or stellate cells. In other embodiments, the microtissues comprise an islet microtissue.

## Experiments and Figures

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Figures

## List of reference numbers

[0097]

10 microfluidic device
11 conduit
12 bend
13 compartment to accommodate a microtissue (also called "microtissue compartment" or "compartment")
13A lower section of compartment adjacent to conduit 13
13B upper section of compartment 13 adjacent to compartment port 62
14 reservoir
15 reservoir port
16 conduit port
17 pillars
41 base plate
42 surface
43 elongated groove which forms part of a conduit
44 liner
61 microtissue
62 compartment port
63 cell comprised in culture liquid
101 lower surface of a microfluidic device
102 upper surface of a microfluidic device
111 relief structure
112 culture liquid
113 pipette
136 inverted microscope
140 frame to accommodate several microfluidic devices
141 platform for rotating or tilting
180 microstructure to create turbulences
160 Lid
161 opening in Lid
162 dome structure
163 Pressure-sensitive adhesive film (100 mm) for sealing of channel structure
166 Frame holder to accommodate multiple frames
140G direction of the force of gravity
Fc centrifugal force

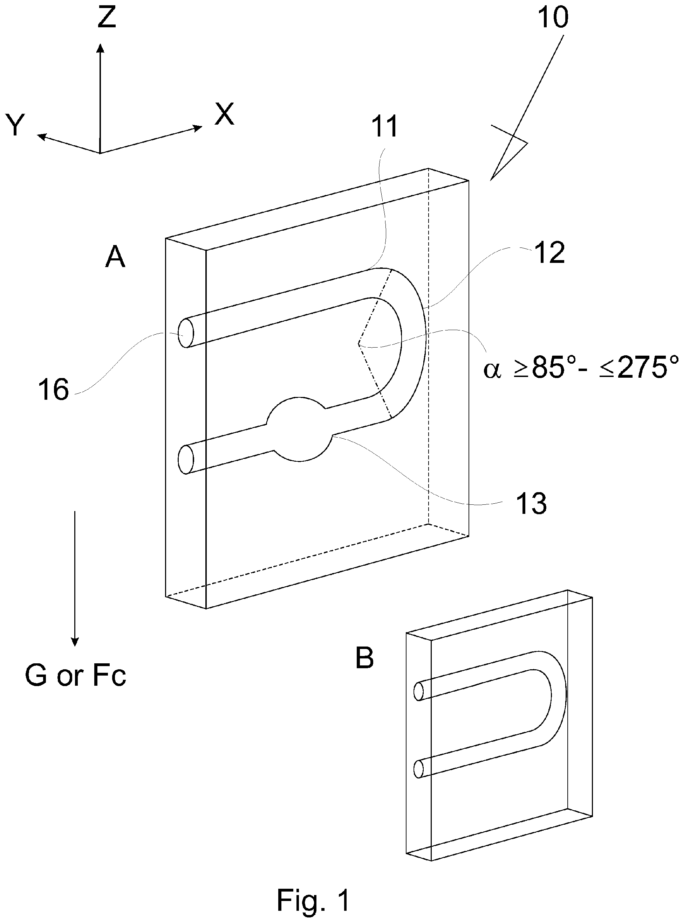

Fig. 1 shows a microfluidic device 10 for accommodation an aqueous liquid, to demonstrate some of the principles of the invention.

In Fig. 1A, the microfluidic device 10 has essentially a planar shape extending into directions X and Z. The device comprises a microfluidic device comprising a conduit 11 fluidically connecting at least two structures selected from compartments, reservoirs and/or ports. The conduit comprises at least one bend 12 describing an angle $\alpha$ of between $\geq 85°$ and $\leq 275°$ around a Y axis arranged orthogonal to the planar shape. The device further comprises a compartment 13 to accommodate a microtissue.

In this embodiment, the open endings of the conduit 11 serve as conduit ports 16, through which culture liquid can for example be loaded into the microfluidic device 10. The port can adopt different technical configurations not shown in this Figure, so as for example to either allow either the insertion of a pipette or the connection of a tube.

The planar shape, or preferably the Z axis thereof, is arranged in an angle of between +45° and -45° relative to the direction of the force of gravity (G), or a centrifugal force (Fc) applied to the microfluidic device.

Fig. 1B shows another embodiment of the invention without the compartment to accommodate a microtissue. It is important to understand that the principle of the invention does also cover embodiments without such a compartment to accommodate a microtissue, which is suitable to study e.g. floating cell interactions.

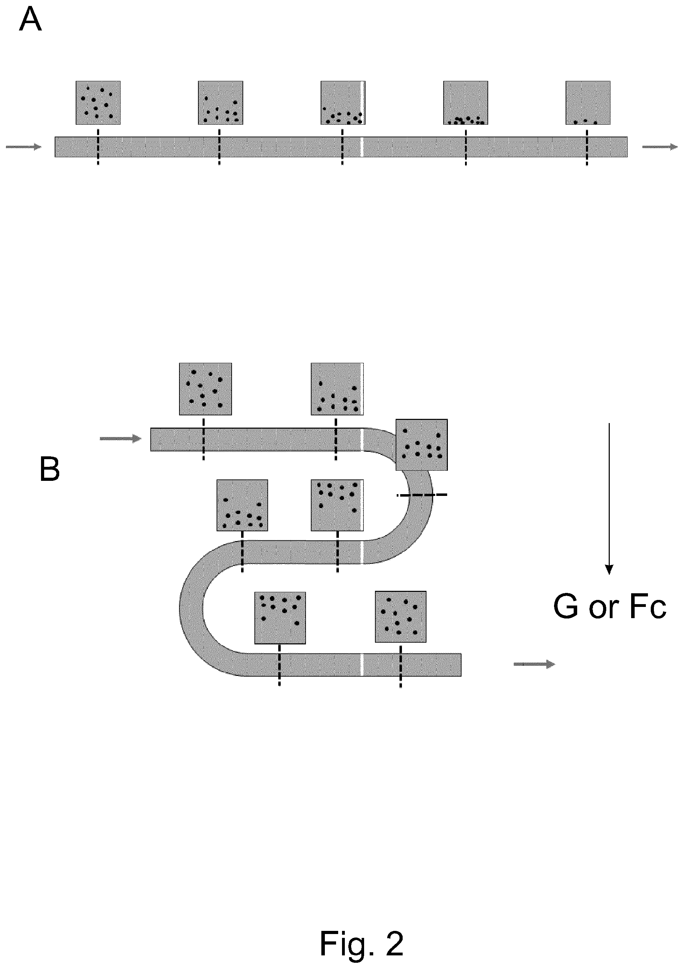

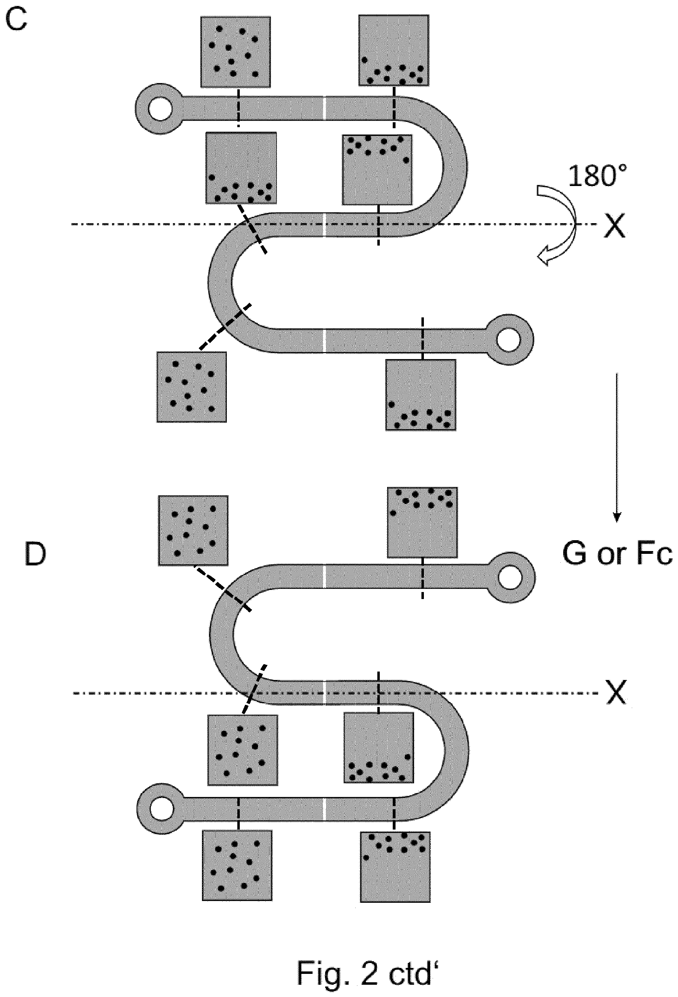

Fig. 2 shows, in a cross section of a microfluidic device according to the invention, the phenomenon of sedimenting cells and/or particles (dots) which are suspended in a liquid medium flowing through a microfluidic conduit (rectangular cross sections) (A, "state of the art"). Due to the relatively small diameter and flow speed, a laminar flow exists causing the cells and/or particles to sediment caused by gravitational or centrifugal forces.

In a microfluidic device according to the invention (B), comprising at least one bend describing an angle $\alpha$ of between $\geq 85°$ and $\leq 275°$ around a Y axis orthogonal to the planar shape sedimentation is avoided, because the lower inner surface of the conduit to which the cells and particles tend to sediment becomes the upper inner surface after a respective bend from which the cells and particles will veer away.

Fig. 2 further shows a rotating concept (C, D) which further contributes to avoiding sedimentation, and furthermore generates a flow of liquid within the conduit. Fig. 2C and Fig. 2D can be seen as a sequence: position 2C is maintained (pause, break) until all liquid has flown from top to bottom and then the device is rotated by 180° to position 2D to induce cells and liquid to flow back. As shown in Fig. 2, a preferred direction of rotation is preferably around the X axis. It can be seen that cells and/or particles that are prone to sedimentation (Fig. 2C, upper right box) are resuspended by the reversal of the relative positioning from top to bottom, which generates a hydrostatically driven reversal of flow through the meander (Fig. 2D, lower right box).

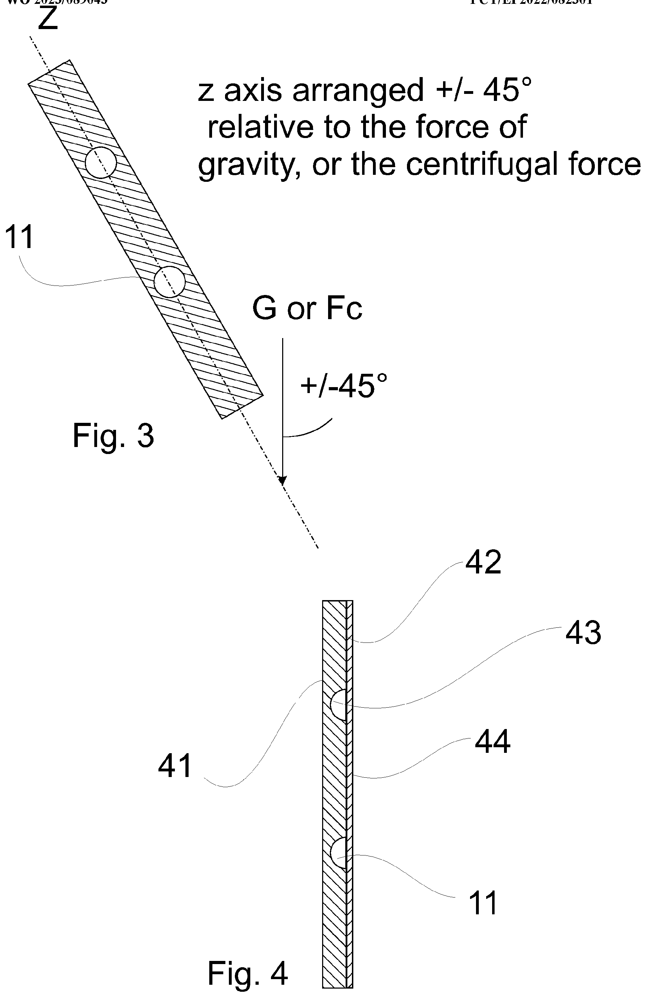

Fig. 3 shows a cross section of the microfluidic device. The planar shape of the microfluidic device, or preferably the Z axis thereof, is arranged in an angle of between +45° and -45° relative to the direction of the force of gravity (G), or a centrifugal force (Fc) applied to the microfluidic device.

Fig. 4 shows a cross section of a microfluidic device according to the invention. The device comprises a base plate 41 with a surface 42 comprising at least one elongated groove 43. On at least part of the respective surface 42 of the base plate 41 a liner 44 is arranged so as to seal the elongated groove. The conduit (as well as the compartments and reservoirs, if present), can be formed, partially or entirely, of the grooves sealed by the liner. The base plate comprising the at least one elongated groove is for example fabricable by milling or molding.

The liner 44 may comprise a foil. The foil may comprise a material selected from the group consisting of polystyrene, PMMA, COC, COP, PTFE, and aluminum, or a mat that may comprise a material selected from a silicone rubber, like e.g. polydimethylsiloxane (PDMS) rubber, vinyl methyl siloxane, phenyl vinyl methyl siloxane, fluorosilicone rubber, and/or nitrile rubber and natural rubber. It may also comprise a foil or mat that is irreversibly bonded to the base plate by e.g. am hotmelt adhesive or an acrylic adhesive.

Because at least one conduit has a polygonal, circular, ellipsoid, semicircular, or semiellipsoid cross section with at least one cross section dimension that is smaller than that of the microtissue, so as to avoid that the microtissue is carried out of the compartment by means of the aqueous liquid.

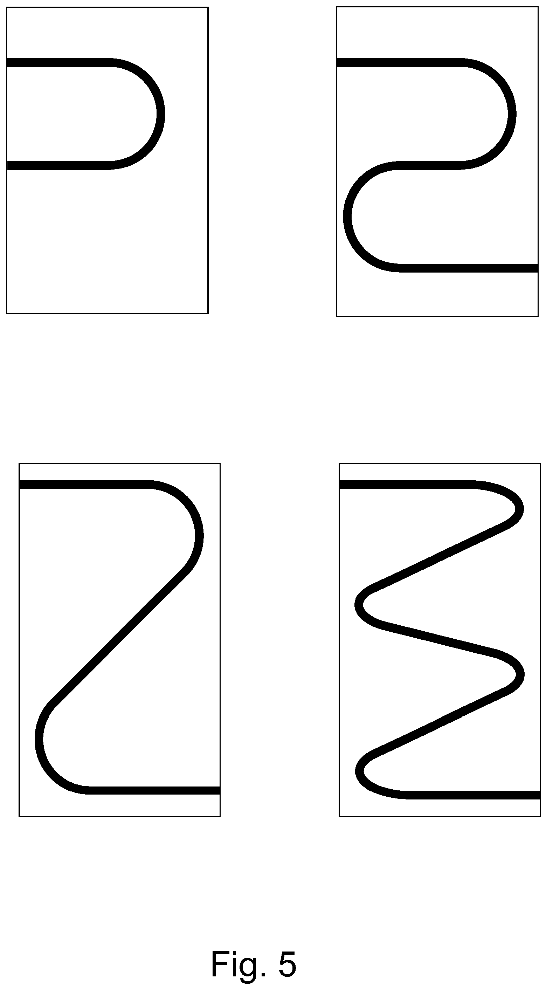

Fig. 5 shows different possible shapes of the conduit, with different numbers of bends and different angles. Compartments, reservoirs and ports are not shown for simplicity.

Fig. 6A shows a view on a microfluidic device with a conduit 11 fluidically connecting at least two structures selected from compartments, reservoirs and/or ports. The conduit comprises at least one bend 12 describing an angle $\alpha$ of between $\geq 85°$ and $\leq 275°$ around a Y axis arranged orthogonal to the planar shape. The device further comprises a series of compartments 13 with compartment ports 62 to accommodate several microtissues. The device further comprises two reservoirs 14 comprising reservoir ports 15. Aqueous liquid optionally comprising cells can be loaded into at least one reservoir via the respective port. A flow of culture liquid is then generated through the conduits and compartments by means of the mechanisms described herein.

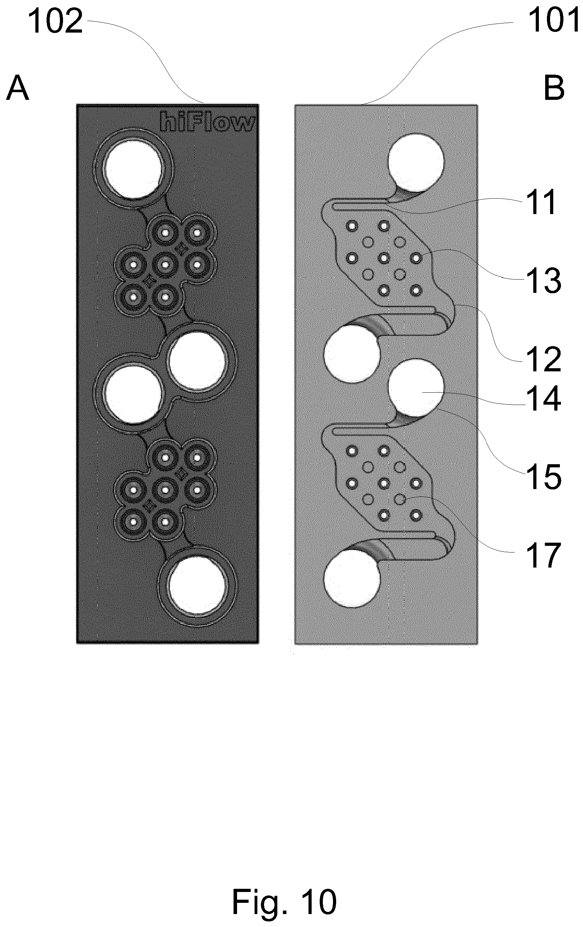

Fig. 6B shows a cross section of another microfluidic device, along line C - C' of Fig 6A, with a conduit 11 fluidically connecting at least two structures selected from compartments, reservoirs and/or ports. The conduit comprises at least one bend not shown in this figure. Fig. 6B shows a compartment 13 to accommodate a microtissue 61, with a compartment port 62 through which the microtissue 61 is loaded into the compartment. Fig. 6B further shows a reservoir 14 comprising reservoir port 15. Aqueous liquid optionally comprising cells 63, e.g., immune cells, can be loaded into the reservoir via the respective reservoir ports 15. A liner 44 is arranged on the lower side of the device to seal the device from the bottom, thus transforming the elongated grooves into conduits 11. Note also that in particular the sizes of the reservoir 14 and the compartment 13 are not drawn to scale - the reservoir 14 is in some embodiments much bigger than the compartment 13 (see, e.g., Fig. 10).

The conduit may have a polygonal, circular, ellipsoid, semicircular, or semiellipsoid cross section with at least one cross section dimension that is smaller than that of the microtissue 61. Hence, the cross-section dimension is selected in such way that microtissues of typical sizes can not pass the conduit, so remain in the compartments.

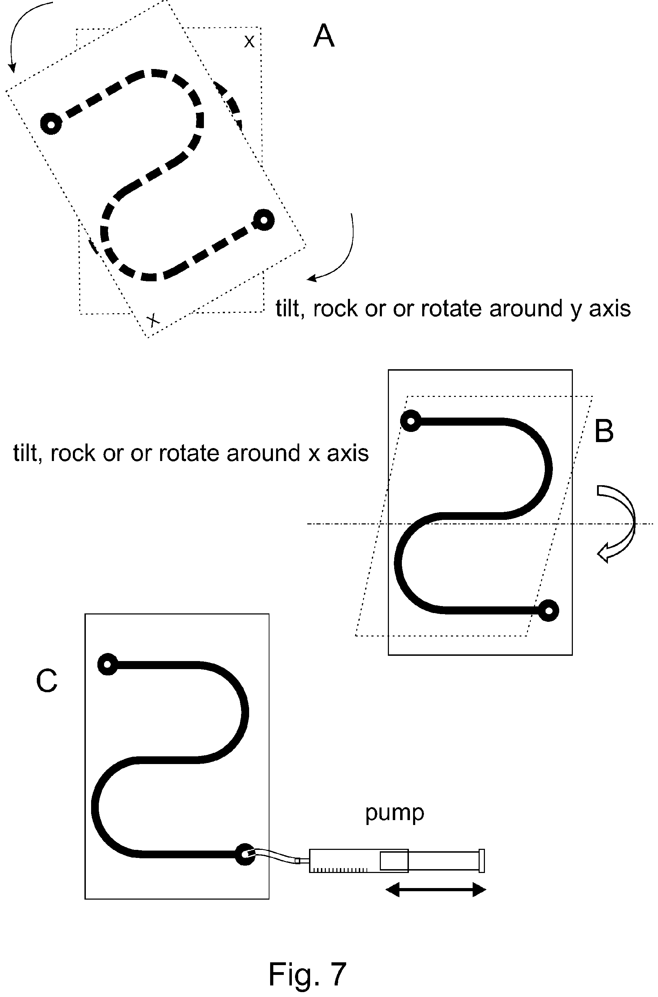

Fig. 7 shows different means to generate a flow of liquid within the conduit. (A) The device can be tilted, rocked back and forth or rotated around the Y axis (preferably by 90°-180°, as shown by the marking "X"). (B) The device can be tilted, rocked back and forth or rotated around the X axis (preferably by 90°-180°). (C) the device can be connected to at least one pump to generate a flow of liquid. Two or three of these approaches can be combined.

With regard to options A) and B), a hydrostatic pressure is generated that drives the flow of liquid. Once all liquid has passed the chip, the tilting has to be repeated.

As can be seen, the device shown in Fig, 7 does not comprise a compartment to accommodate a microtissue. While the tilting principle shown in Fig. 7 applies to all embodiments of the present in invention, in particular with and without a compartment to accommodate a microtissue, it is important to understand that the principle of the invention does also cover embodiments without such a compartment to accommodate a microtissue, which is suitable to study e.g. floating cell interactions.

Fig. 8 shows different arrangements of conduits 11, microtissue compartments 13, reservoirs 14 reservoir ports 15 and compartment ports 62. In one embodiment, several compartments and conduits are arranged in parallel to one another. In another embodiment, the conduit widens up and accommodates the compartmentsP. It can be seen that in these and other embodiments, the reservoirs have, generally, a greater diameter than the microtissue compartments.

In Fig. 8A and 8B, the compartments 13 are arranged between two sections of the conduit 11. In Fig. 8B, the conduit furcates into two or more conduits which are arranged in parallel, in which each furcated conduit comprises at least one compartment arranged between two sections thereof.

In Fig 8C the compartments 13 are arranged within the conduit 11, i.e., the conduit 11 widens up and accommodates the compartments 13

Fig. 9 shows examples of cross sections of the conduits. The conduits can have a polygonal, circular or ellipsoid cross section. The conduits can also consist of elongated grooves having an open ended semicircular, semiellipsoid or rectangular cross section. In such case, they are sealed with a liner shown as a dotted line to produce the conduits.

Fig. 10 shows one embodiment of a microfluidic device according to the invention.

Fig 10 A and B show a plan view of a microfluidic device according to the invention. Figs 10 C and D show a slanted view of a microfluidic device according to the invention.

Fig. 10 B and D show a lower surface 101 of the microfluidic device with conduits 11, bends 12, compartments 13 and reservoirs 14. The lower surface 101 can be sealed by means of a liner 44 (not shown), which is preferably transparent to allow microscopic inspection of *inter alia* the compartments and the microtissues comprised therein.

Fig. 10 A and C show an upper surface 102 of the microfluidic device. The reservoirs 14 comprise rims which serve as reservoir ports 15 through which culture liquid, optionally comprising cells, can be loaded. The compartments 13 comprise rims which serve as compartments ports 62, through which microtissues 61 (not shown) can be loaded.

The area in which the compartments 13 are located (in this embodiment, 7 compartments are provided per area), pillars 17 are provided (in this embodiment, 4 pillars are provided per area), to provide a constant distance between the liner and the upper surface of the area, so as to make sure that the space provided by the compartments 13 for the microtissues 61 is constant.

Hence, in Fig. 10, the compartments 13 are arranged within the conduit 11, i.e., the conduit 11 widens up and accommodates the compartments 13. The device as shown in Fig. 10 provides permanent access to the cells and medium and is suitable to be used, directly, on a microscope, in particular an inverted microscope and/or a confocal microscope.

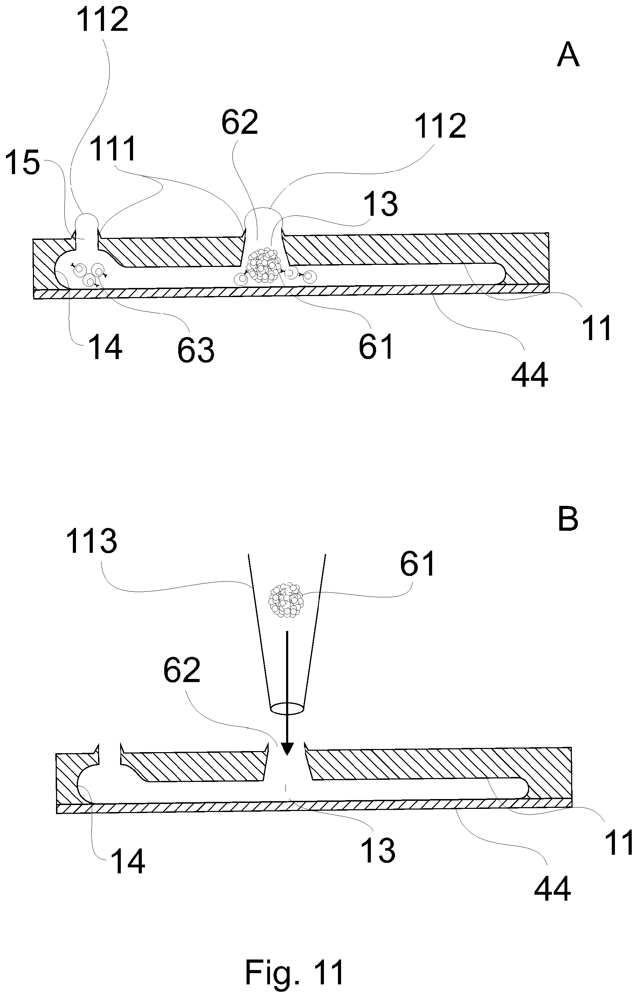

Fig. 11 A shows a cross section of a microfluidic device according to the invention, with a conduit 11 fluidically connecting at least two structures selected from compartments, reservoirs and/or ports. The conduit comprises at least one bend not shown in this figure. Fig. 11 shows a compartment 13 to accommodate a microtissue 61, with a compartment port 62 through which the microtissue 61 is loaded into the compartment. A liner 44 is arranged on the lower side of the device to seal, inter alia, the reservoir, conduit and compartment. Fig. 11 further shows a reservoir 14 comprising reservoir port 15. Aqueous liquid optionally comprising cells 63, e.g., immune cells, can be loaded into the reservoir via the respective reservoir ports 15, for example to interact with the microtissue 61.

The reservoir port 15 and the compartment port 62 comprise relief structures 111 (not drawn to scale), which are exemplarily shown in the form of an overflow-preventing edge, which allow that droplets 112 of culture liquid form at the top of the ports, by establishing a maximum contact angle, thus preventing release of the droplet by capillary pinning. This phenomenon increases the size of the liquid-air interface, and hence improves the gas exchange between the culture liquid and a surrounding gaseous medium. The droplets are drawn in an exaggerated fashion. Note also that in particular the sizes of the reservoir 14 and the compartment 13 are not drawn to scale - the reservoir 14 is in some embodiments much bigger than the compartment 13 (see, e.g., Fig. 10).

It is to be mentioned that the relief structure 111 can adopt different shapes, with different angles, heights, and roundings, as long as the shape fulfills its purpose, as described above. The relief structure can alternatively comprise a non-wettable area (i.e., comprising a hydrophobic area, or an area equipped with lotus effect), to achieve the same effect.

Further, the relief structure avoids spilling of the liquid when the device is tilted, or when a pressure is applied in the microfluidic device, e.g., by pumping activity from the reservoirs with liquid flow through the channels. In both situations, the droplet that forms at the top of the well may increase in volume, or swell, but will not spill or overflow. In this aspect, the surface tension of liquid, which creates a pressure barrier, has a contributory effect.

Instead of the overflow-preventing edge, such relief structure can also comprise a non-wettable area (i.e., comprising a hydrophobic area, or an area equipped with lotus effect).

The relative sizes of reservoir 14 and microtissue compartment 13 are not to scale. In many embodiments, the reservoirs 14 have a greater volume than the mictrotissue compartments 13.

Fig. 11 B shows a cross section of a microfluidic device according to the invention, with a conduit 11 fluidically connecting at least two structures selected from compartments, reservoirs and/or ports. The conduit comprises at least one bend not shown in this figure. Fig. 11 shows a compartment 13 to accommodate a microtissue 61, with a compartment port 62 through which the microtissue 61 is loaded into the compartment by means of a pipette 113. A liner 44 is arranged on the lower side of the device to seal, inter alia, the reservoir, conduit and compartment.

The device as shown in Fig. 11 provides permanent access to the cells and medium and is suitable to be used, directly, on a microscope, in particular an inverted microscope and/or a confocal microscope.

Fig. 12A shows a top view of a microfluidic device 10 according to the invention. Fig. 12B shows, in enlarged fashion, the area around a compartment 13 to accommodate a microtissue 61. Fig. 12C shows a cross section of that area. The compartment has a circular cross section, and has a compartment port 62 with a circular cross section, arranged concentrically to the compartment. The absolute dimensions given are just exemplary and not binding. The relief structure 111 is optional.

In one embodiment, the compartment has, in its lower section 13A, a dome structure 162, to reduce the risk that a microtissue 61 comprised in the compartment is washed out during operation of the microfluidic device. Tests have shown that, relative to a lower section that has a conical or funnel shaped tapering towards the compartment port 62 the dome structure 162 significantly reduces such risk. Note that the dome structure is not drawn to scale n this image. Preferably, it ends at the intersection of lower section 13A and upper section 13B. See Fig. 16C which better reflects the real dimensions.

The diameter of the compartment 13 to accommodate a microtissue is d1, the diameter of the compartment port is d2, and the diameter of the microtissue is d3. In one embodiment, $(d1-d2)/2 > d3$. In such way, it is made sure that the microtissue does not escape through the compartment port.

The device as shown in Fig. 12 provides permanent access to the cells and medium and is suitable to be used, directly, on a microscope, in particular an inverted microscope and/or a confocal microscope.

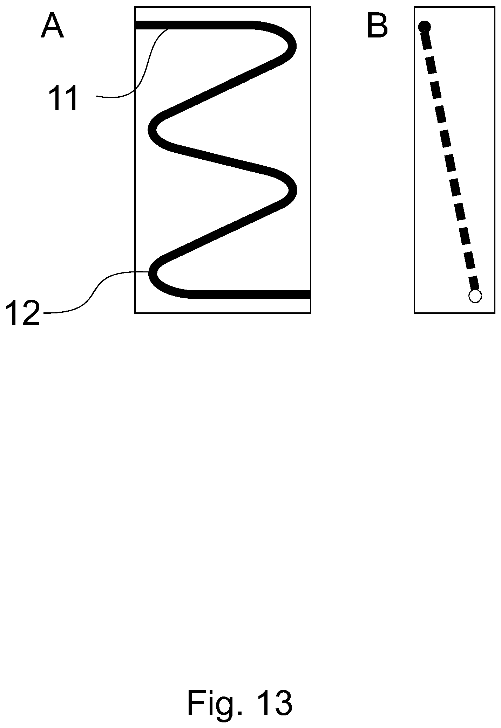

Fig 13A shows a view of a simplified device according to the invention, with a conduit 11 and bends 12. Fig. 13B shows a cross section thereof, demonstrating that in this embodiment, the conduit is not arranged in parallel to the surface of the device, but strides across the device in a plane relatively inclined to the surface thereof.

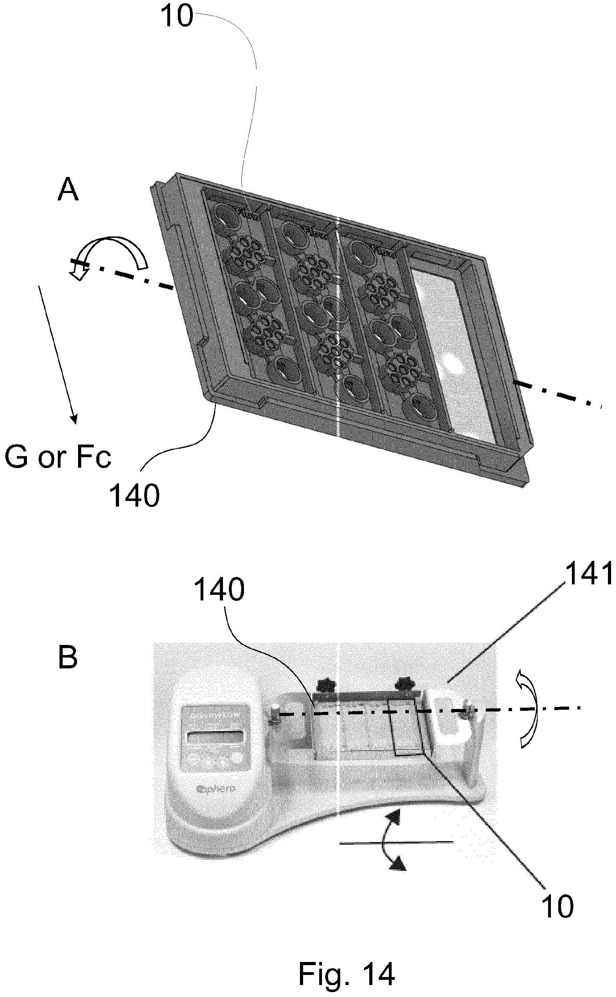

Fig. 14 A shows an array of several microfluidic devices 10 according to the invention, arranged on a frame 140 that is suitable to be rotated. The microfluidic devices can be fastened on the frame by means of snap-in connectors, yet other suitable fastening means are also possible (including velcro or magnetic plates).

Fig. 14 B shows a platform 141 that is capable of rotating or tilting microfluidic devices 10 arranged on a frame 140.

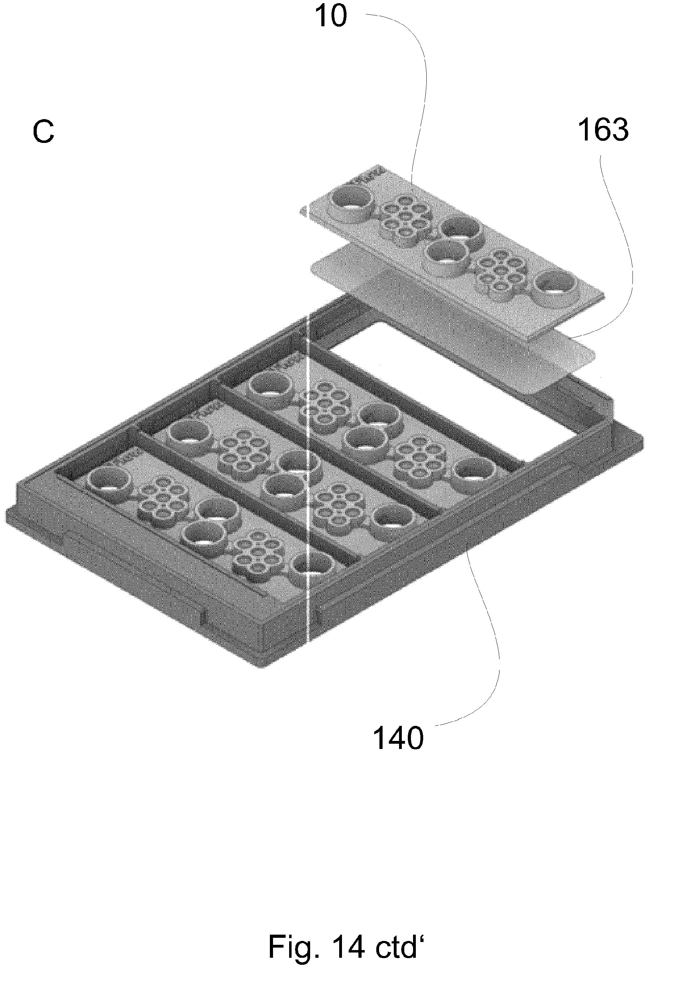

Fig. 14 C shows another variant of the device shown in Fig. 14A The microfluidic device 10 comprises an injection-molded polystyrene body and a pressure-sensitive adhesive film 163 (with e.g. 100 mm thickness) for sealing of the channel structure of the microfluidic device 10. The frame 140 accomodates an assembly/array of 4 such microfluidic devices and adopts a standard SLAS/ANSI format.

The injection mold process and the standardized format allow pre-series mass-fabrication of reproducible devices with low production effort and in high quantities. It also allows the use of inert martial with low compound adsorption, and allows reliance on validated surface coating protocols and chemistry to achieve cell repellent properties.

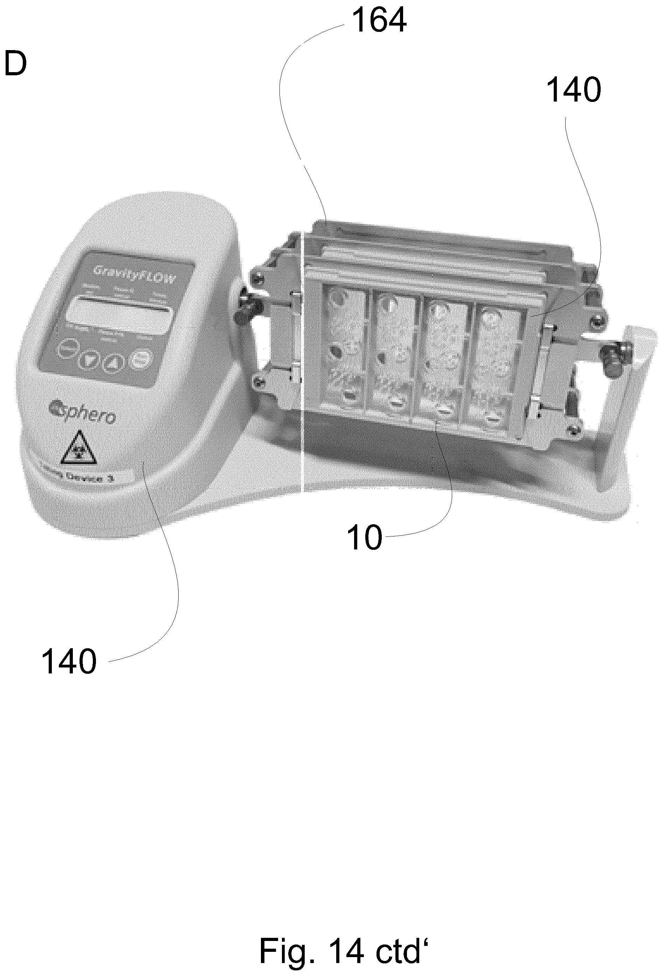

Fig. 14 D shows a platform 141 that is capable of rotating or tilting microfluidic devices 10 arranged on a frame 140. The microfluidic devices 10 are stacked in multiple frames 140 arranged on a frame holder 164. In such way, a large number of experiments can be carried out in parallel by stacking multiple frames. A bi-directional perfusion of the microfluidic devices 10 is accomplished through periodic, vertical tilting, which recirculates single cells in suspension.

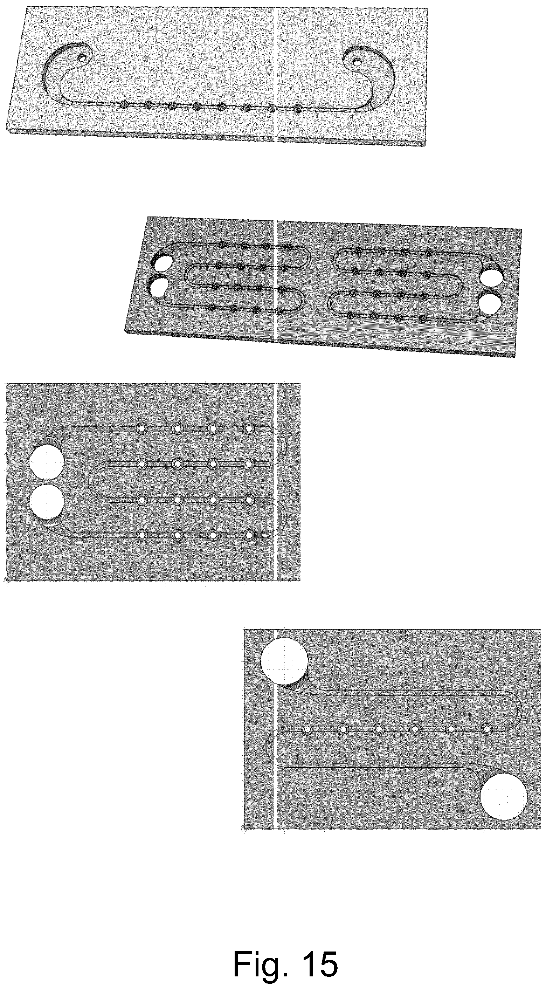

Fig. 15 shows different examples of a microfluidic device according to the present invention.

Fig. 16 A and B show cross sections of a microfluidic device 10 according to the invention, with the reservoir ports 15, the compartments 13 to accommodate a microtissue and the compartment ports 62. A liner 44 is barely visible, yet seals the elongated grooves, and hence forms the conduit 11. The device can be placed e.g. on an inverted microscope 136 to allow tissue inspection.

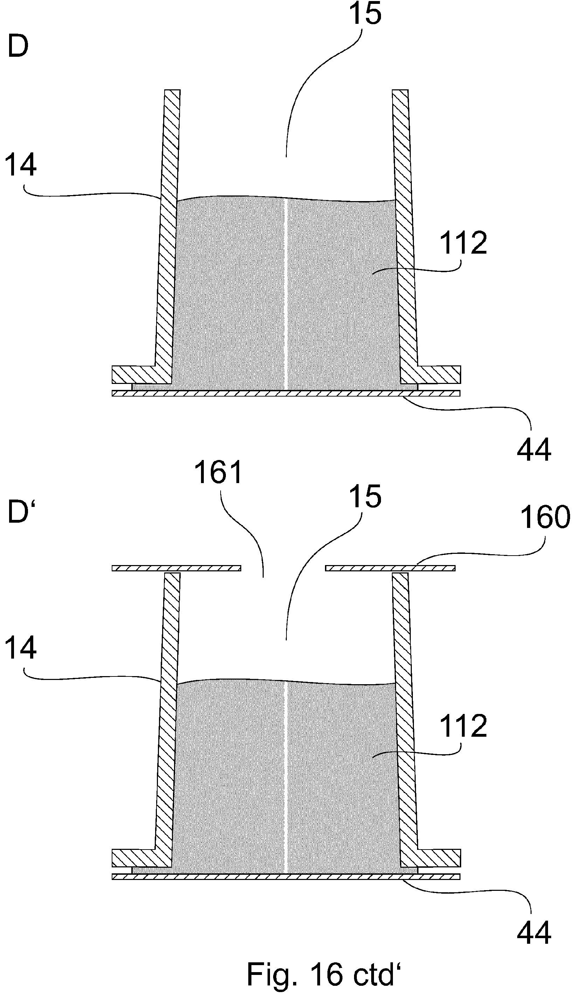

Fig 16 C and D show, as cross sections, close-ups of the microtissue compartment (Fig. 16C) and the reservoir (Fig. 16D and D') of a microfluidic device according to the invention.

The microtissue compartment comprises a lower section 13A and an upper section13B. The upper section 13B comprises a compartment port 62 with a relief structure 111 which avoids spilling of the culture liquid 112. A lid 160 is provided which adopts the shape of a foil sealing the dice on its upper section, so as to avoid spilling or loss of culture liquid by evaporation, as well as contamination from above. The lower section comprises a dome structure 162, to reduce the risk that a microtissue 61 comprised in the compartment is washed out during operation of the microfluidic device. Tests have shown that, relative to a lower section that has a conical or funnel shaped tapering towards the compartment port 62 the dome structure 162 significantly reduces such risk. As discussed above, preferably, the upper end of the dome structure defines the beginning of the upper section.

The reservoir 14 comprises a reservoir port 15 that is likewise sealed with a lid 160, which comprises an opening 161 to allow addition of culture liquid 112. Preferably, the lid has a circular shape, and is concentrically arranged to the reservoir and the reservoir port, which likewise have a circular shape.

Fig 16 E and F show, as cross sections, close-ups of the microtissue compartment (Fig. 16E) and the reservoir (Fig. 16F) of a microfluidic device according to the invention, when said device is arranged in operational mode, i.e., when its Z-axis is parallel to the direction of gravity or centrifugal force.

In the microtissue compartment, spilling of culture liquid 112 is avoided by the relief structure, which is provided in the form of an overpinning edge, namely through surface tension or capillary pinning of the liquid and coherent forces between the liquid and the surface of the relief structure. Note that, instead of the overflow-preventing edge, such relief structure can also comprise a non-wettable area (i.e., comprising a hydrophobic area, or an area equipped with lotus effect).

In the reservoir, no such relief structure is provided, yet spilling of culture liquid is avoided due to the lid opening 161 being concentrically arranged to the reservoir and the reservoir port.

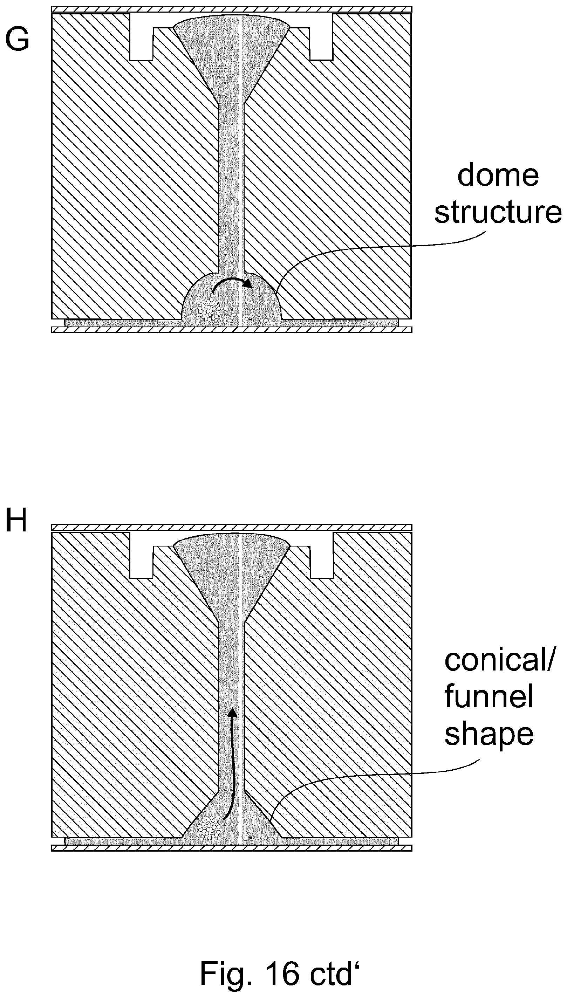

Fig. 16G shows again an embodiment with a dome structure 162, to reduce the risk that a microtissue 61 comprised in the compartment is washed out during operation. Tests have shown that, relative to a lower section that has a conical or funnel shaped tapering towards the compartment port 62 the dome structure 162 can significantly reduce such risk.

Fig. 16H shows for comparison only, an embodiment not according to the invention, with a lower section that has a conical or funnel shaped tapering towards the compartment port 62. It can be seen that in such embodiment, the microtissue 61 comprised in the compartment is easily washed out during operation.

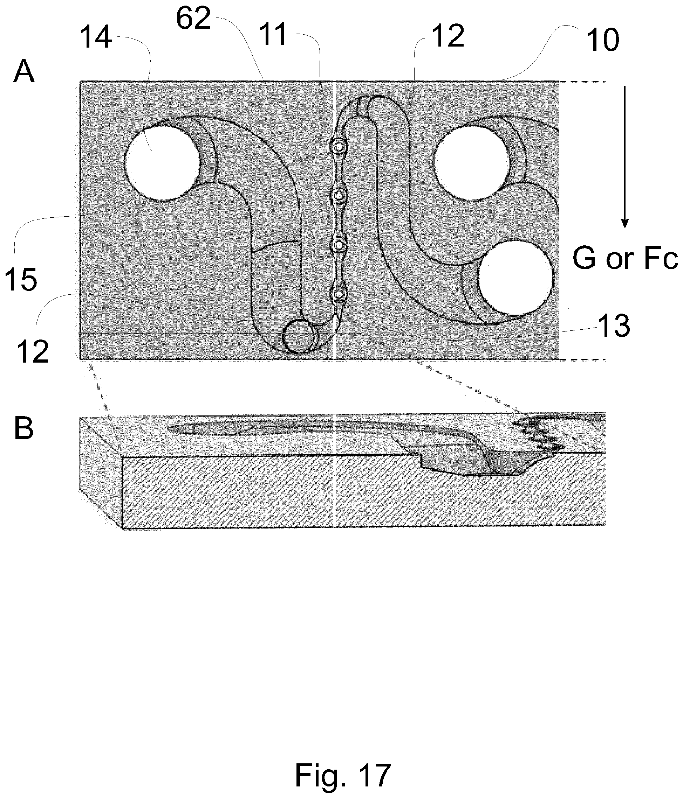

Fig. 17 A shows a bottom view of a microfluidic device 10 according to the invention, with the liner 44 left away for clarity, with the reservoirs 14 comprising reservoir ports 15, the conduit 11 comprising bends 12, and compartments 13 to accommodate microtissues, comprising compartment ports 62. The liner to seal the elongated grooves and thus forming the conduits is not shown.

Fig. 17 B shows a cross section of the same microfluidic device to illustrate the profile of the ports, compartments and conduits.

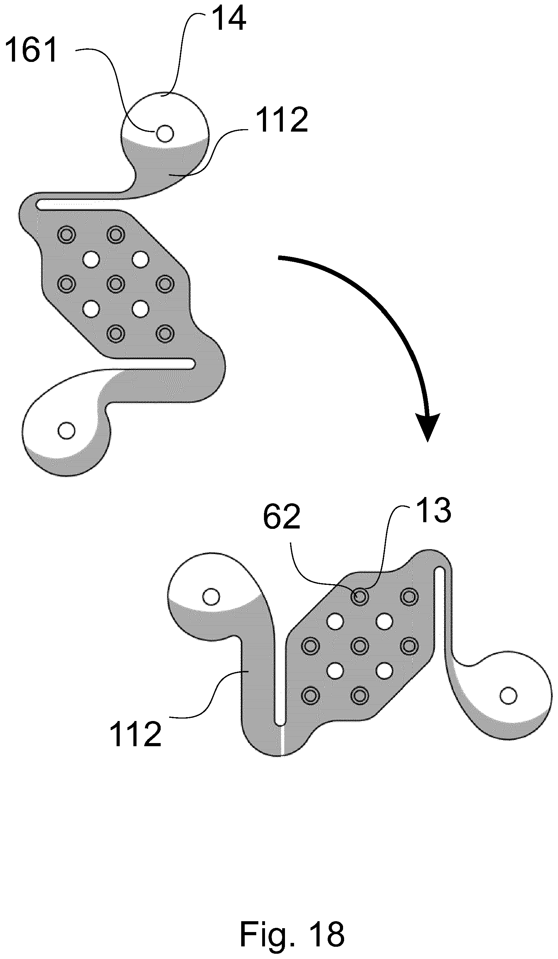

Fig. 18 shows a view of a microfluidic device according to the invention in two different operational mode positions, i.e., with its Z-axis parallel and orthogonal to the direction of gravity or centrifugal force. In the reservoir 14, spilling of culture liquid 112 is avoided in either of the two positions, representative to any of the operating positions between 0 and +/-180°, due to the lid opening 161 being concentrically arranged to the reservoir 14 and the reservoir port 15.

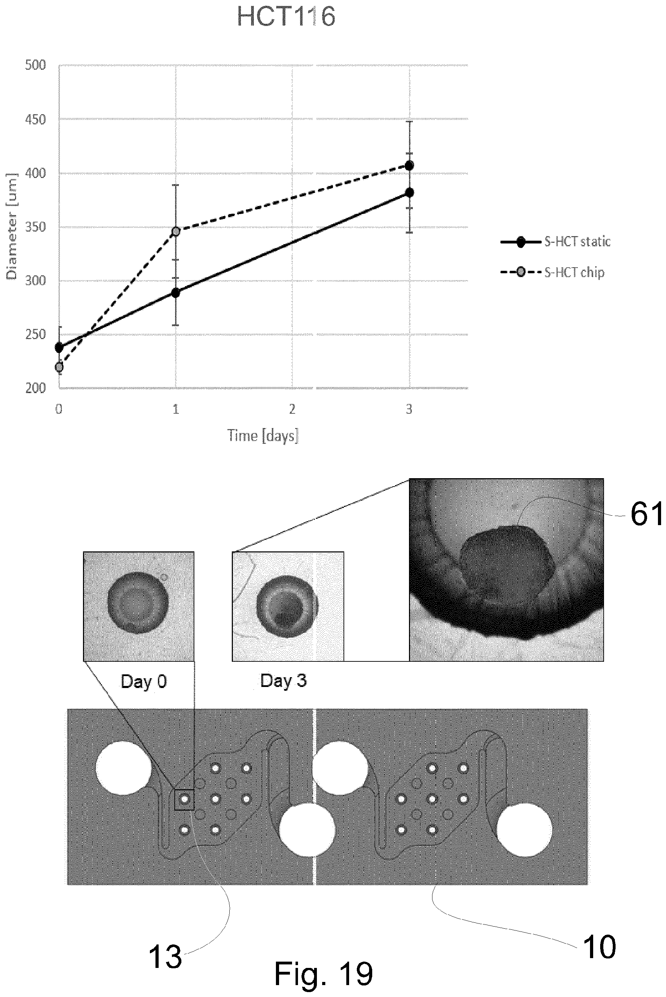

Fig. 19 shows tumor growth in the microtissue compartment. HCT116 tumor microtissues 61 were grown in compartments 13 of the microfluidic device 10 over 3 days and have shown similar growth as under static conditions.

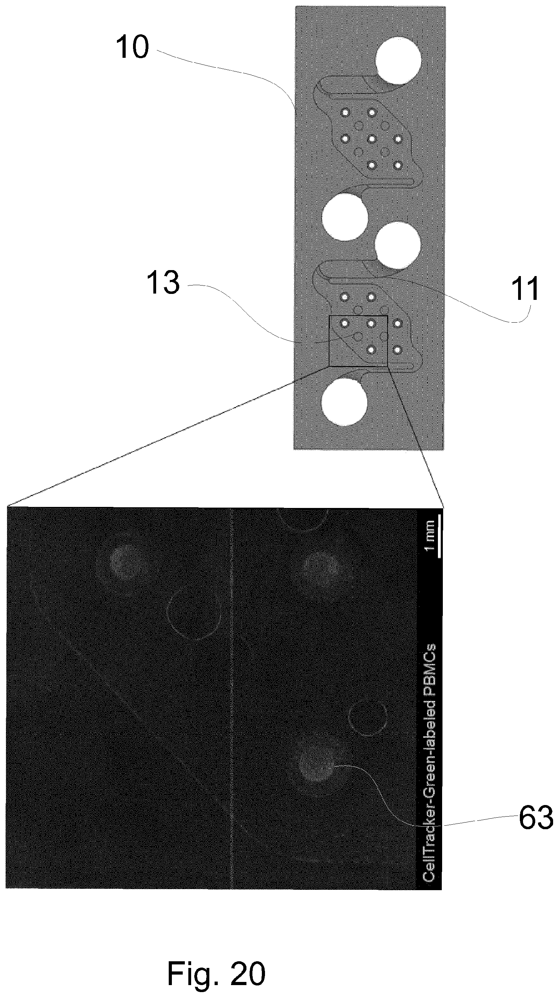

Fig. 20 shows PBMC distribution in the compartments 13. The distribution of immune cells 63 (Peripheral Blood Mononuclear Cells, "PBMC") within the compartments 13 of the microfluidic device 10 was assessed by labelling the cells with a fluorescent dye and monitoring them under perfusion conditions with a handheld fluorescent microscope. The cells moved freely through the conduits 11 and the compartments 13.

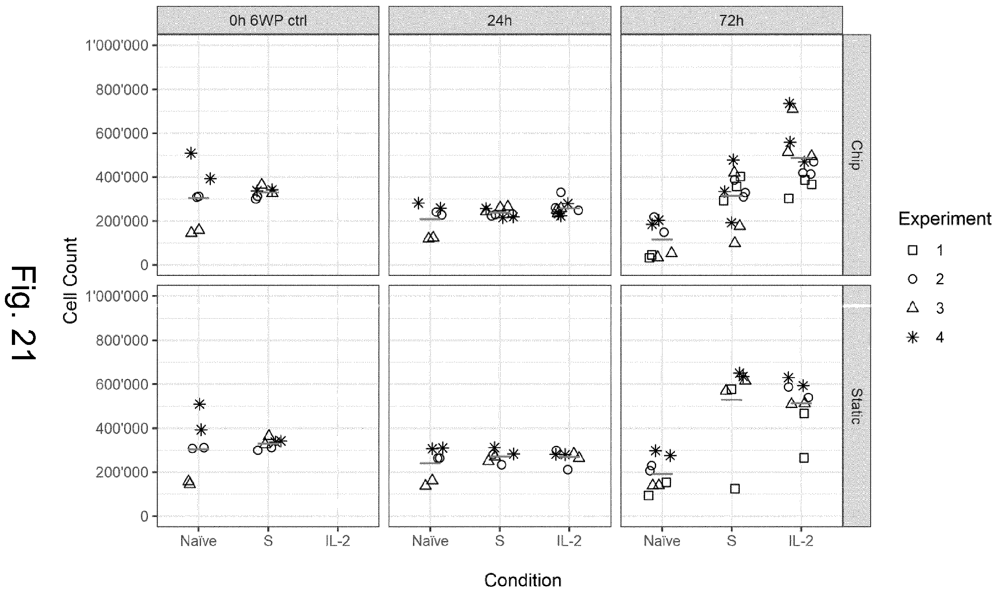

Fig. 21 shows a cell count of PBMCs, as measured with a precision count beads by flow cytometry. Human primary PBMCs in naïve or stimulated conditions were loaded onto the microfluidic device according to the present invention (called "Chip" herein), or 96-well ULA plates (Akura™ 96 spheroid microplates, InSphero AG, called "Static" herein). The *0h 6WP ctrl* measurement shows the cell count that was transferred into the microfluidic device or onto the plates. The cells were harvested from the microfluidic device after 24 h and 72 h of culturing. The cell count shows a representative number of cells being harvested and processed for flow cytometry from the microfluidic device system and the static well-plate control. Proliferation was observed for stimulated cells after 72 h under perfusion and static conditions.

Results obtained with microfluidic device according to the present invention were comparable to control conditions cultured in 96-well ULA pates. The results indicate that no sedimentation and clumping occurred over the time course of the experiment, when cells were cultured in the microfluidic device and could be easily removed. The results of four independent experiments show the robustness of cell retrieval from the microfluidic device for downstream analysis.

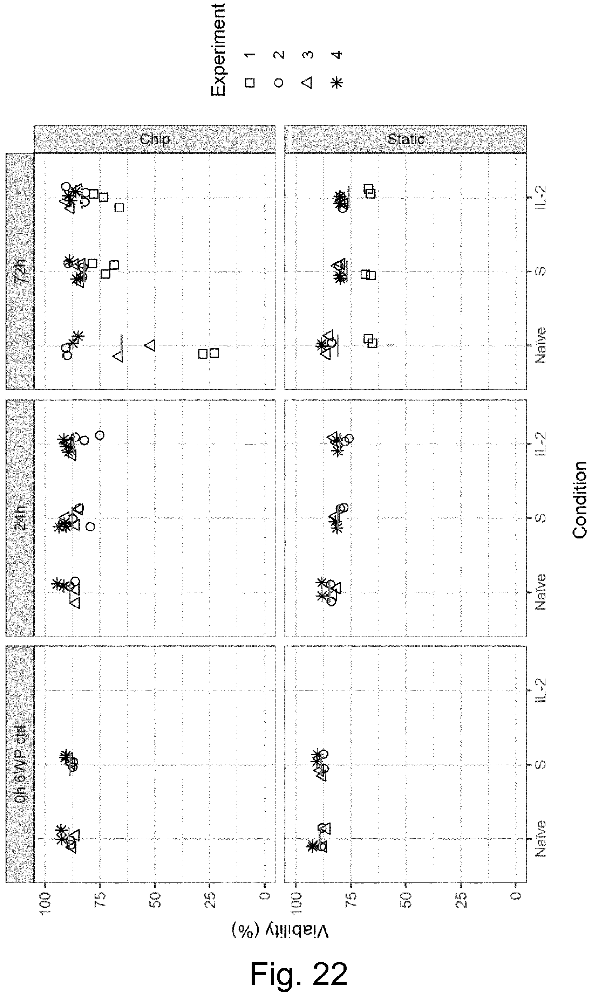

Fig. 22 presents viability of PBMCs in different conditions over time. Viability of PBMCs, as measured with a fixable viability stain by flow cytometry. Human primary PBMCs in naïve or stimulated conditions were loaded onto the microfluidic device according to the present invention, or 96-well ULA plates. The *0h 6WP ctrl* measurement shows the viability at the time of transfer. The cells were harvested from the microfluidic device after 24 h and 72 h of culturing. Viability assessment showed high viability over for at least 72 h of culturing. Results obtained with the microfluidic device according to the present invention were comparable to control conditions cultured in 96-well ULA pates. The results of four independent experiments show the robustness of the culturing conditions and the suitability of the microfluidic device for PBMC culturing.

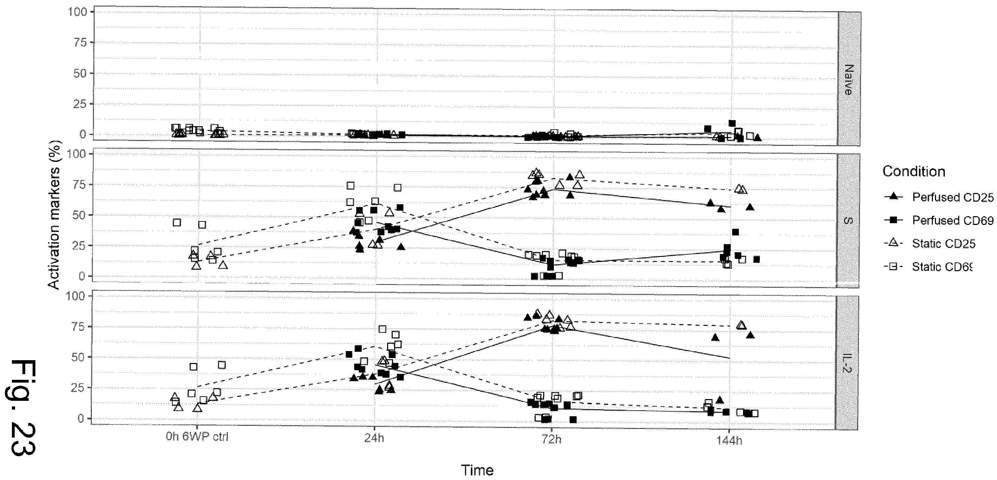

Fig. 23 shows activation Markers of Cytotoxic T cells. Phenotyping of cytotoxic (CD3+/CD8+) T cells for their activation status. Naive and stimulated (S and IL-2) PBMCs were cultured for up to 144 h under perfusion and static culturing conditions. Cells were harvested after 24 h, 72 h, and 144 h and analyzed by flow cytometry. Early activation markers (CD69) showed highest expression on stimulated T cells after 24 h. Late activation markers (CD25) were highly expressed after 72 h and 144 h. Naive cells showed no expression of T cell activation markers.

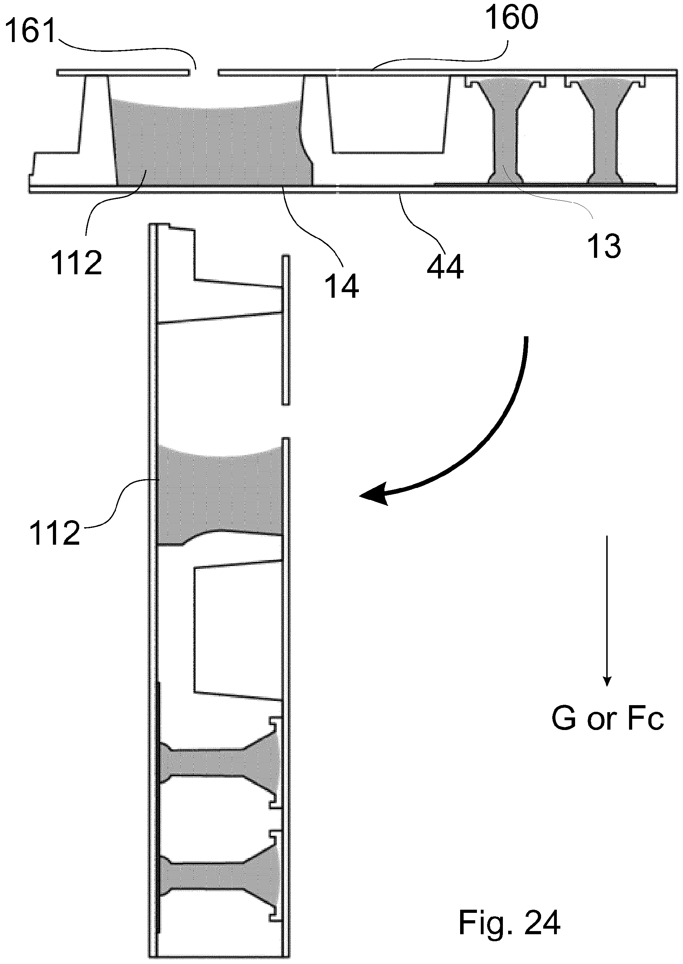

Fig. 24 shows a cross section of a microfluidic device according to the invention in a level position and in a position in operational mode, i.e., with its Z-axis parallel to the direction of gravity or centrifugal force. In the microtissue compartment 13, spilling of culture liquid 112 is avoided by the relief structure, which is provided in the form of an overpinning edge, namely through surface tension or capillary pinning of the liquid and coherent forces between the liquid and the surface of the relief structure. Note that, instead of the overflow-preventing edge, such relief structure can also comprise a non-wettable area (i.e., comprising a hydrophobic area, or an area equipped with lotus effect). In the reservoir 14, no such relief structure is provided, yet spilling of culture liquid is avoided due to the lid opening 161 being concentrically arranged to the reservoir and the reservoir port.

Figs. 26 - 28 show experimental results with primary human PBMCs. Cells were thawed and cultured in ultra-low adhesion 6-well plates. After 24 h of resting, they were harvested, counted and transferred into the microfluidic device (perfused) at different cell densities in a total volume of 300 mL per channel (conduit). Static controls were loaded into Akura™ 96 plates at the same densities and a total volume of 100 mL per well. After three and six days of culturing, cells were harvested from the device or plates respectively and were processed for flow cytometry analysis.

Fig. 26 shows the results of PMBC culturing on chip and cell count and viability. Cells were mixed with counting beads to determine the number of harvested cells, and they were stained with fixable viability stain (FVS575V, BD Biosciences) for viability measurement. Data was analyzed in a BD Fortessa, (BD Biosciences) equipped with a High Throughput Sampler. Data analysis was performed in FlowJo (BD Biosciences) by gating singlets, then separating cellular events and bead events in the forward scatter and side scatter. Viable cells were unstained for FVS575V. As a result, the viability of PBMC is generally high under both, static and perfused conditions.

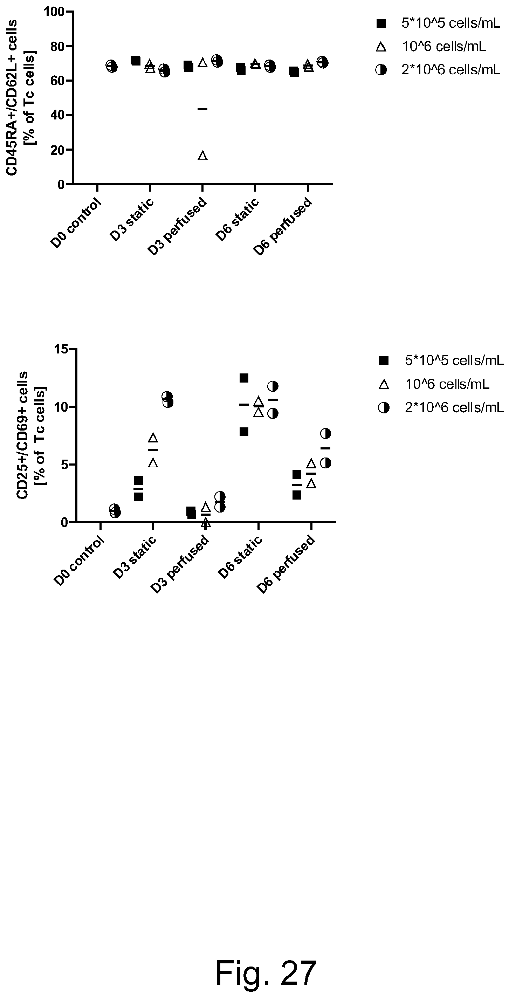

Fig. 27 shows the results of T-cell activation experiments. Cells were mixed with counting beads to determine the number of harvested cells, and they were stained with fixable viability stain (FVS575V) for viability measurement and a panel of surface marker antibodies to determine T cells and their activation states. Cells were activated with CD3/CD28. Data was analyzed in a BD Fortessa equipped with a High Throughput Sampler. Viable cytotoxic T cells (Tc cells; FVS575V-, CD3+, CD8+, CD4-) were analyzed and the fraction of naive Tc cells (CD45RA+, CD62L+) and activated Tc cells (CD25+; CD69+) were determined. As a result, similar levels of naïve Tc cells in all conditions were obtained, yet slightly more pronounced density-dependence of activated Tc cells under static culturing conditions.

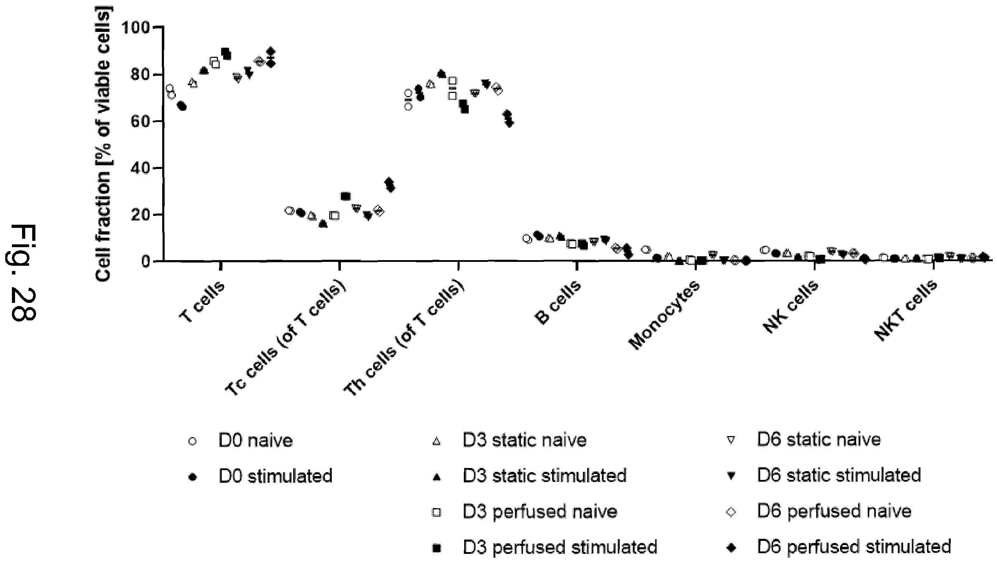

Fig. 28 shows the cellular composition. Cells were mixed with counting beads to determine the number of harvested cells, and they were stained with fixable viability stain (FVS575V) for viability measurement and a panel of surface marker antibodies to determine and quantify the different cellular subpopulations of PBMCs. Data was analyzed in a BD Fortessa equipped with a High Throughput Sampler. Cells were determined as T cells (CD3+, CD56-), cytotoxic T cells (Tc cells; CD3+, CD56-, CD8+, CD4-), helper T cells (Th cells; CD3+, CD56-, CD4+, CD8-), B cells (CD3-, CD19+), Monocytes (CD3-, CD14+), natural killer cells (NK cells; CD3-, CD56+), natural killer T cells (NKT cells; CD3+, CD56+). As a result, similar cell composition was obtained under all culturing condition, with Tc cell fraction larger and Th cell fraction smaller under stimulated perfusion condition.

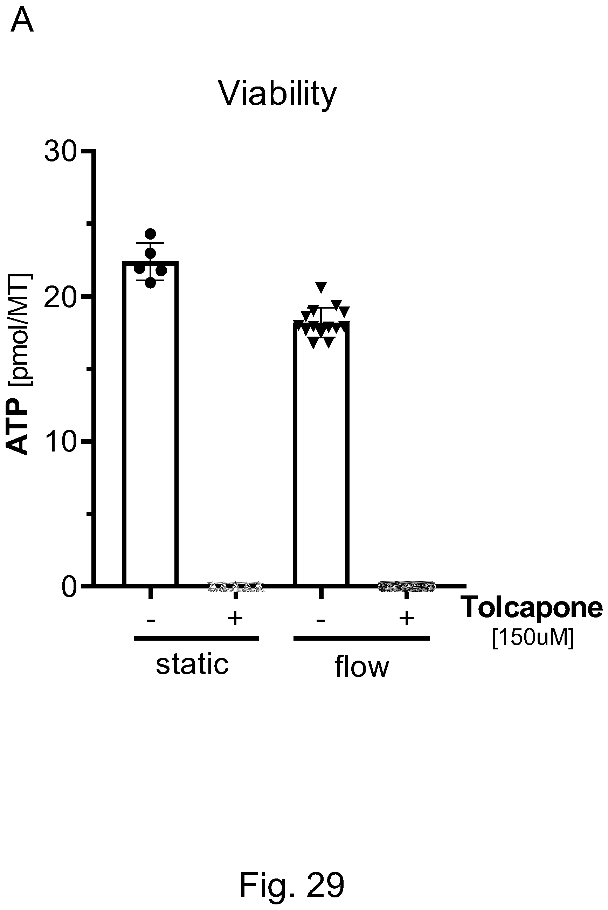

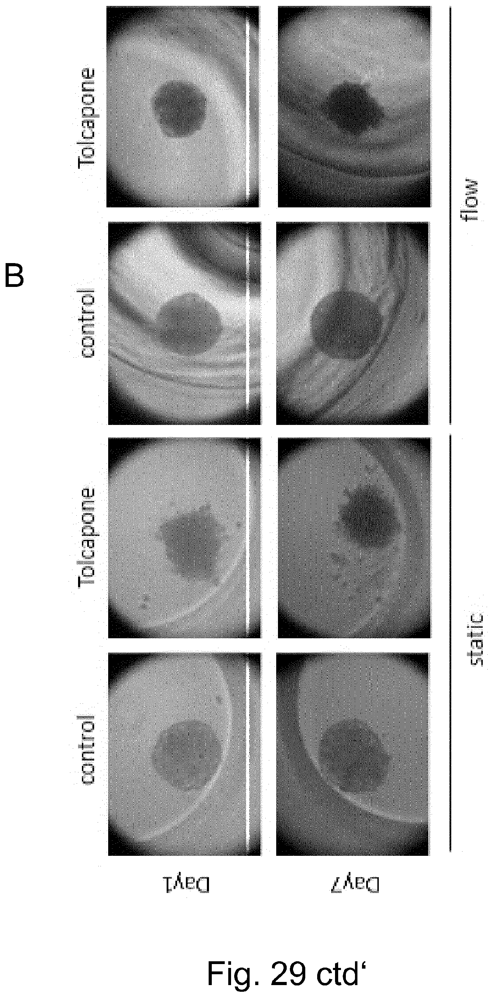

Fig. 29 shows results of experiments with a 3D liver model. The viability and the response to known drug effect have been tested in the microfluidic device and compared to static conditions in a well plate. Primary liver microtissues have been loaded into the dedicated compartments of the microfluidic device and cultured under perfusion (flow) over 7 days. Medium exchange has been performed at day 2 and 6 after loading. Brightfield images were acquired at day 1 and day 7. At day 7, microtissues were retrieved from the microfluidic device and the ATP content - correlating to tissue viability - was determined using Promega's CellTiter GLO kit. In parallel, microtissue were cultured in well plates under static condition using the same protocol. Microtissues cultured under flow as well as under static conditions showed high viability and healthy morphology after 7 days. Microtissues exposed to 150 mM Tolcapone resulted in complete loss of ATP and thus could be considered as not viable anymore, as expected. These results are supported by the injured morphology of the tissues.

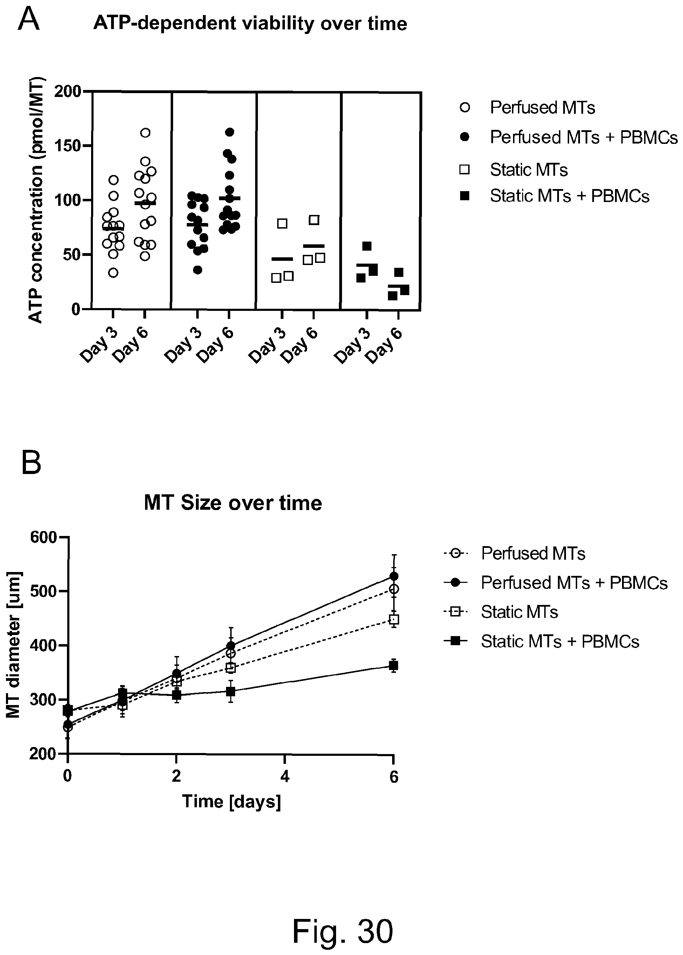

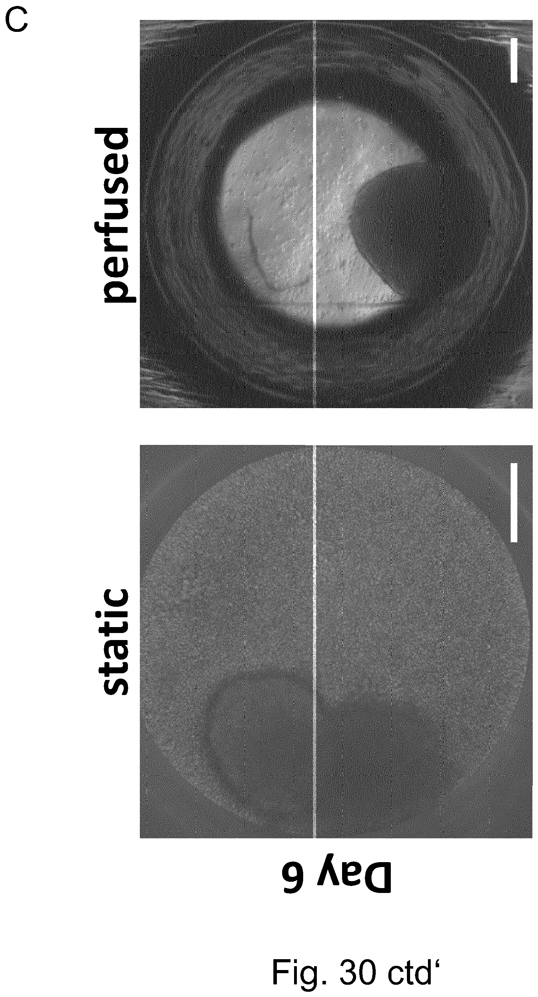

Fig. 30 shows results of an experiment with HCT116 colon cancer cell microtissues. HCT116 colon cancer cell microtissues (MTs) were transferred into a microtissue device comprising seven compartments per channel (conduit) (Perfused MTs). After 24 h of resting, PBMCs were added to the medium reservoirs of the channels (conduit) and distributed throughout the channels (conduit) (Perfused MTs + PBMCs). Bright field images of the MTs were taken daily for size measurements and the viability of MTs was determined by intracellular ATP measurement after three days and six days of co-culture. Static control conditions were cultured in Akura™96 plates at same PBMC densities and one MT per well (Static MTs, Static MTs + PBMCs). As a result, MTs grow faster under perfusion conditions and feature higher ATP concentrations than under static conditions. Presence of naive PBMCs had no observable effect on growth behavior of MTs. PBMCs visible in MT compartments, no clustering and no aggregations of PBMCs around MTs under perfusion conditions.

## References

- Ruppen, J. et al. A microfluidic platform for chemoresistive testing of multicellular pleural cancer spheroids. Lab Chip 14, 1198-1205 (2014).
- Occhetta P, Centola M, Tonnarelli B, Redaelli A, Martin I, Rasponi M. High-Throughput Microfluidic Platform for 3D Cultures of Mesenchymal Stem Cells, Towards Engineering Developmental Processes. Sci Rep. 2015 May 18;5:10288
- Kwapiszewska K, Michalczuk A, Rybka M, Kwapiszewski R, Brzózka Z. A microfluidic-based platform for tumour spheroid culture, monitoring and drug screening. Lab Chip. 2014 Jun 21; 14(12):2096-104
- Jin, H.-J., Cho, Y.-H., Gu, J.-M., Kim, J., Oh, Y.-S., 2011. A multicellular spheroid formation and extraction chip using removable cell trapping barriers. Lab Chip 11,115-119, http://dx.doi.org/10.1039/c01c00134a.
- Hsiao, A. Y. et al. Microfluidic system for formation of PC-3 prostate cancer co-culture spheroids. Biomaterials 30, 3020-3027 (2009).
- Torisawa, Y. et al. A multicellular spheroid array to realize spheroid formation, culture, and viability assay on a chip. Biomaterials 28, 559-566 (2007).
- Wu, L. Y., Di Carlo, D. & Lee, L. P. Microfluidic self-assembly of tumour
- spheroids for anticancer drug discovery. Biomed. Microdevices 10, 197-202
- (2008).
- Messner S, Agarkova I, Moritz W, Kelm JM. Multi-cell type human liver microtissues for hepatotoxicity testing. Arch Toxicol. 2013 Jan;87(1):209-13. doi: 10.1007/s00204-012-0968-2. Epub 2012 Nov 11. PMID: 23143619; PMCID: PMC3535351.

## Claims

**1.** A microfluidic device (10) suitable for accommodating an aqueous liquid, the device having essentially a planar shape, which device comprises at least one conduit (11) fluidically connecting at least two structures selected from reservoirs (14) and/or ports (15, 62),

wherein said conduit comprises at least one bend (12) describing an angle of between ≥ 85° and ≤ 275° around an axis orthogonal to the planar shape
which planar shape is arranged in an angle of between +45° and -45° relative to the direction of

a) the force of gravity (G), or
b) a centrifugal force (Fc) applied to the microfluidic device.

**2.** The microfluidic device according to claim 1, which further comprises at least one compartment (13) arranged within the conduit, or between two sections thereof, said compartment being capable of accommodating at least one microtissue.

**3.** The microfluidic device according to any one of claims 1 - 2, wherein at least one compartment (13) has a circular cross section, and has a compartment port (62) with a circular cross section arranged concentrically to the compartment (13), and wherein the diameter of the compartment is larger than the diameter of the compartment port.

**4.** The microfluidic device according to any one of claims 1 - 3, wherein at least one conduit has a polygonal, circular, ellipsoid, semicircular, or semiellipsoid cross section with at least one cross section dimension smaller than 250 μm.

**5.** The microfluidic device to any one of claims 1 - 4, which device further comprises, or is connected to or mountable on, a means to generate a flow of aqueous liquid within the conduit, preferably wherein the means to generate a flow of liquid is selected from the group consisting of a tilting device, a tiltable device, a rotating device and a pump.

**6.** The microfluidic device according to any one of claims 1 - 5, wherein the reservoir and/or conduit is suitable to accommodate a liquid optionally comprising cells and/or particles.

7. The microfluidic device according to any one of claims 1 - 6, wherein at least one port is suitable to be connected to an external tube or hose, which tube or hose is optionally connected to a pump.

8. The microfluidic device according to any one of claims 1 - 7, wherein the conduit has two ends each of which fluidically connects to a reservoir, preferably wherein at least one reservoir comprises a port.

9. The microfluidic device according to any one of claims 1 - 8, which device comprises a base plate (41) with a surface (42) comprising at least one elongated groove (43), wherein on at least part of the respective surface (42) of the base plate (41) a liner (44) is arranged so as to seal the elongated groove, and hence form the conduit (11).

10. The microfluidic device according to claim 9, wherein the liner (44) comprises a foil or a mat.

11. An assembly or array comprising a plurality of microfluidic devices according to any one of claims 1 - 10.

12. The assembly or array according to claim 11, which comprises at least one tilting unit, tiltable unit, rotating unit and/or a pump.

13. A method of culturing at least one microtissue and/or at least one cell in a microfluidic device according to any one of claims 1 - 10, which method comprises,

   • loading at least one microtissue and/or at least one cell into the microfluidic device, and, simultaneously, before or subsequently, introducing an aqueous liquid into the microfluidic device
   • establishing a liquid flow through the microfluidic device,

wherein, due to the bend provided in at least one conduit (11), the sedimentation of non-soluble components in the aqueous liquid is avoided.

14. The method according to claim 13, wherein the liquid flow is driven or supported by at least one of:

   • a pump, and/or
   • tilting, turning, rotating, pivoting or rotating the device.

15. The method according to any one of claims 13 or 14, wherein the aqueous liquid comprises cells and/or particles.

**Patentansprüche**

1. Mikrofluidische Vorrichtung (10), die geeignet ist, eine wässrige Flüssigkeit aufzunehmen, und die im Wesentlichen eine ebene Form aufweist, wobei die Vorrichtung mindestens eine Leitung (11) aufweist, die mindestens zwei Strukturen, die aus Behältern (14) und/oder Öffnungen (15, 62) ausgewählt sind, fluidisch verbindet, wobei die Leitung mindestens eine Biegung (12) aufweist, die einen Winkel zwischen

   $\geq 85°$ und $\leq 275°$ um eine Achse orthogonal zur ebenen Form beschreibt,
   wobei die ebene Form in einem Winkel zwischen +45° und -45° angeordnet ist, relativ zu der Richtung

   a) der Schwerkraft (G), oder
   b) einer Zentrifugalkraft (Fc), die auf die mikrofluidische Vorrichtung wirkt.

2. Mikrofluidische Vorrichtung nach Anspruch 1, die ferner mindestens ein Kompartiment (13) aufweist, das innerhalb der Leitung oder zwischen zwei Abschnitten derselben angeordnet ist, wobei das Kompartiment mindestens ein Mikrogewebe aufnehmen kann.

3. Mikrofluidische Vorrichtung nach einem der Ansprüche 1 bis 2, wobei mindestens ein Kompartiment (13) einen kreisförmigen Querschnitt und eine konzentrisch zum Kompartiment (13) angeordnete Kompartimentöffnung (62) mit kreisförmigem Querschnitt aufweist, und wobei der Durchmesser des Kompartiments größer ist als der Durchmesser der Kompartimentöffnung

4. Mikrofluidische Vorrichtung nach einem der Ansprüche 1 bis 3, wobei mindestens eine Leitung einen polygonalen, kreisförmigen, ellipsoiden, halbkreisförmigen oder halbellipsoiden Querschnitt mit mindestens einer Querschnittsabmessung kleiner als 250 $\mu$m aufweist.

5. Mikrofluidische Vorrichtung nach einem der Ansprüche 1 bis 4, wobei die Vorrichtung ferner ein Mittel zum Erzeugen eines Stroms einer wässrigen Flüssigkeit innerhalb der Leitung aufweist oder damit verbunden oder daran montierbar ist, wobei das Mittel zum Erzeugen eines Flüssigkeitsstroms vorzugsweise aus der Gruppe ausgewählt ist bestehend aus einer Kippvorrichtung, einer kippbaren Vorrichtung, einer rotierenden Vorrichtung und einer Pumpe.

6. Mikrofluidische Vorrichtung nach einem der Ansprüche 1 bis 5, wobei das Reservoir und/oder die Leitung geeignet ist, eine Flüssigkeit aufzunehmen, die optional Zellen und/oder Partikel aufweist.

7. Mikrofluidische Vorrichtung nach einem der Ansprüche 1 bis 6, wobei mindestens eine Öffnung zum Anschluss an einen externen Schlauch oder eine externe Röhre geeignet ist, wobei der Schlauch oder die Leitung optional mit einer Pumpe verbunden ist.

8. Mikrofluidische Vorrichtung nach einem der Ansprüche 1 bis 7, wobei die Leitung zwei Enden aufweist, die jeweils fluidisch mit einem Reservoir verbunden sind, wobei vorzugsweise mindestens ein Reservoir eine Öffnung aufweist.

9. Mikrofluidische Vorrichtung nach einem der Ansprüche 1 bis 8, wobei die Vorrichtung eine Grundplatte (41) mit einer Oberfläche (42) aufweist, die mindestens eine längliche Rille (43) aufweist, wobei auf mindestens einem Teil der jeweiligen Oberfläche (42) der Grundplatte (41) eine Schicht (44) angeordnet ist, um die längliche Rille abzudichten und somit den Kanal zu bilden (11).

10. Mikrofluidische Vorrichtung nach Anspruch 9, wobei die Schicht (44) eine Folie oder eine Matte aufweist.

11. Baugruppe oder Anordnung aufweisend eine Vielzahl von mikrofluidischen Vorrichtungen nach einem der Ansprüche 1 bis 10.

12. Baugruppe oder Anordnung nach Anspruch 11, die mindestens eine Kippeinheit, eine kippbare Einheit, eine Dreheinheit und/oder eine Pumpe aufweist.

13. Verfahren zur Kultivierung mindestens eines Mikrogewebes und/oder mindestens einer Zelle in einer mikrofluidischen Vorrichtung nach einem der Ansprüche 1 bis 10, wobei das Verfahren aufweist,

   • Einbringen mindestens eines Mikrogewebes und/oder mindestens einer Zelle in die mikrofluidische Vorrichtung und gleichzeitiges, vor oder nach dem Einbringen, Einleiten einer wässrigen Flüssigkeit in die mikrofluidische Vorrichtung
   • Herstellen eines Flüssigkeitsstroms durch die mikrofluidische Vorrichtung,

   wobei aufgrund der Krümmung, die in mindestens einer Leitung (11) vorgesehen ist, die Sedimentation von nicht löslichen Komponenten in der wässrigen Flüssigkeit vermieden wird.

14. Verfahren nach Anspruch 13, wobei der Flüssigkeitsstrom durch mindestens eines der folgenden Elemente angetrieben oder unterstützt wird:

   • eine Pumpe, und/oder
   • Kippen, Drehen, Rotieren, Schwenken oder Rotieren der Vorrichtung.

15. Verfahren nach einem der Ansprüche 13 oder 14, wobei die wässrige Flüssigkeit Zellen und/oder Partikel aufweist.

## Revendications

1. Dispositif microfluidique (10) apte à recevoir un liquide aqueux, le dispositif ayant essentiellement une forme plane, lequel dispositif comprend au moins un conduit (11) reliant à fluide au moins deux structures sélectionnées parmi des réservoirs (14) et/ou des orifices (15, 62),

   dans lequel ledit conduit comprend au moins un coude (12) décrivant un angle compris entre $\geq$ 85° et $\leq$ 275° autour d'un axe orthogonal à la forme plane,
   laquelle forme plane est agencée selon un angle compris entre +45° et -45° par rapport à la direction

      a) de la force de gravité (G), ou
      b) d'une force centrifuge (Fc) appliquée au dispositif microfluidique.

2. Dispositif microfluidique selon la revendication 1, qui comprend en outre au moins un compartiment (13) agencé à l'intérieur du conduit, ou entre deux sections de celui-ci, ledit compartiment étant capable de recevoir au moins un microtissu.

3. Dispositif microfluidique selon l'une quelconque des revendications 1 à 2, dans lequel au moins un compartiment (13) présente une section transversale circulaire, et comporte un orifice de compartiment (62) ayant une section transversale circulaire agencée de façon concentrique par rapport au compartiment (13), et dans lequel le diamètre du compartiment est plus grand que le diamètre de l'orifice de compartiment.

4. Dispositif microfluidique selon l'une quelconque des revendications 1 à 3, dans lequel au moins un conduit présente une section transversale polygonale, circulaire, ellipsoïdale, semi-circulaire ou semi-ellipsoïdale avec au moins une dimension de section transversale étant plus petite que 250 $\mu$m.

5. Dispositif microfluidique selon l'une quelconque des revendications 1 à 4, lequel dispositif comprend en outre, ou est relié à ou pouvant être monté sur, un moyen pour produire un écoulement de liquide aqueux à l'intérieur du conduit, de préférence dans lequel le moyen pour produire un écoulement de liquide est sélectionné dans le groupe constitué par un dispositif d'inclinaison, un dispositif inclinable, un dispositif rotatif et une pompe.

6. Dispositif microfluidique selon l'une quelconque des revendications 1 à 5, dans lequel le réservoir et/ou le conduit est apte à recevoir un liquide comprenant de manière optionnelle des cellules et/ou des particules.

7. Dispositif microfluidique selon l'une quelconque des revendications 1 à 6, dans lequel au moins un orifice est apte à être relié à un tube ou tuyau externe, lequel tube ou tuyau est de manière optionnelle relié à une pompe.

8. Dispositif microfluidique selon l'une quelconque des revendications 1 à 7, dans lequel le conduit présente deux extrémités dont chacune est reliée à fluide à un réservoir, de préférence dans lequel au moins un réservoir comprend un orifice.

9. Dispositif microfluidique selon l'une quelconque des revendications 1 à 8, lequel dispositif comprend une plaque de base (41) avec une surface (42) comprenant au moins une rainure allongée (43), dans lequel sur au moins une partie de la surface respective (42) de la plaque de base (41) un revêtement (44) est agencé de manière à sceller la rainure allongée, et formant ainsi le conduit (11).

10. Dispositif microfluidique selon la revendication 9, dans lequel le revêtement (44) comprend une feuille ou une natte.

11. Un ensemble ou réseau comprenant une pluralité de dispositifs microfluidiques selon l'une quelconque des revendications 1 à 10.

12. L'ensemble ou réseau selon la revendication 11, qui comprend au moins une unité d'inclinaison, une unité inclinable, une unité rotative et/ou une pompe.

13. Un procédé de culture d'au moins un microtissu et/ou d'au moins une cellule dans un dispositif microfluidique selon l'une quelconque des revendications 1 à 10, lequel procédé comprend :

• le chargement d'au moins un microtissu et/ou d'au moins une cellule dans le dispositif microfluidique, et de façon simultanée, avant ou par la suite, l'introduction d'un liquide aqueux dans le dispositif microfluidique
• l'établissement d'un écoulement de liquide à travers le dispositif microfluidique,

dans lequel, grâce au coude prévu dans au moins un conduit (11), la sédimentation de composants non solubles dans le liquide aqueux est évitée.

14. Procédé selon la revendication 13, dans lequel l'écoulement de liquide est entraîné ou favorisé par au moins l'un des moyens suivants :

• une pompe, et/ou
• l'inclinaison, le basculement, la rotation, le pivotement ou la rotation du dispositif.

15. Procédé selon l'une quelconque des revendications 13 ou 14, dans lequel le liquide aqueux comprend des cellules et/ou des particules.

Fig. 1

Fig. 2

C

180°

X

G or Fc

D

X

Fig. 2 ctd'

z axis arranged +/- 45°
relative to the force of
gravity, or the centrifugal force

G or Fc

+/-45°

Fig. 3

Fig. 4

Fig. 5

Fig. 6

Fig. 6 ctd'

A

tilt, rock or or rotate around y axis

tilt, rock or or rotate around x axis

B

C

pump

Fig. 7

Fig. 8

Fig. 9

Fig. 10

102

62

15

C

D

11

101

12

14

13

Fig. 10 ctd'

Fig. 11

EP 4 433 572 B1

Fig. 12

30

A

11

12

B

Fig. 13

Fig. 14

C

10

163

140

Fig. 14 ctd'

164

D

140

140

10

Fig. 14 ctd'

Fig. 15

15

10

A

62

111

13

62

10

B

13B

13A

13

44    11

100x/1,25

136

Fig. 16

Fig. 16 ctd'

Fig. 16 ctd'

Fig. 16 ctd'

G

dome
structure

H

conical/
funnel
shape

Fig. 16 ctd'

Fig. 17

Fig. 18

Fig. 19

Fig. 20

Fig. 21

Fig. 22

Fig. 23

Fig. 24

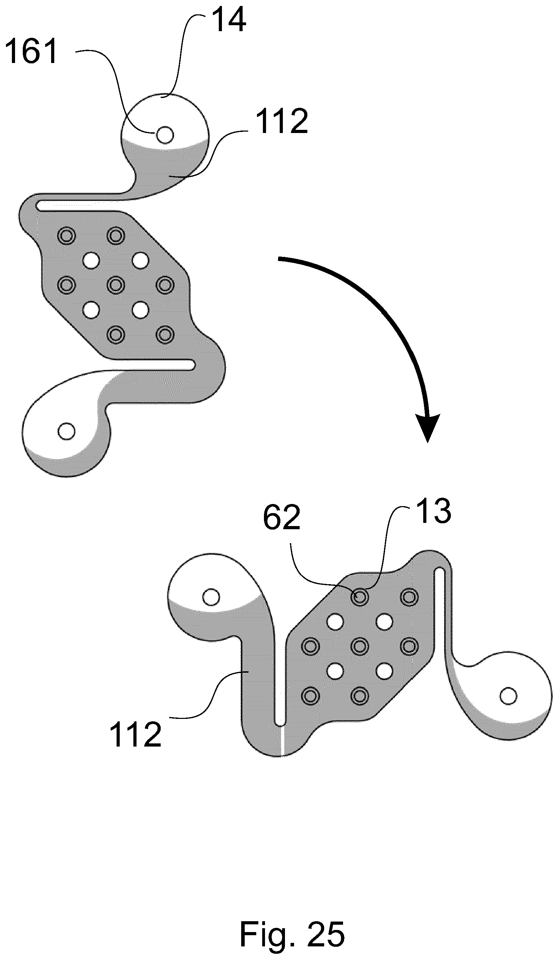

Fig. 25

Fig. 26

Fig. 27

Fig. 28

A

Fig. 29

Fig. 29 ctd'

## A   ATP-dependent viability over time

○  Perfused MTs

●  Perfused MTs + PBMCs

□  Static MTs

■  Static MTs + PBMCs

## B

### MT Size over time

-○-  Perfused MTs

-●-  Perfused MTs + PBMCs

-□-  Static MTs

-■-  Static MTs + PBMCs

## Fig. 30

C

perfused

static

Day 6

Fig. 30 ctd'

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006037033 A
- CA 3142903
- US 2019009274 A

**Non-patent literature cited in the description**

- **RUPPEN, J et al.** A microfluidic platform for chemoresistive testing of multicellular pleural cancer spheroids.. *Lab Chip*, 2014, vol. 14, 1198-1205
- **OCCHETTA P** ; **CENTOLA M** ; **TONNARELLI B** ; **REDAELLI A** ; **MARTIN I** ; **RASPONI M.** High-Throughput Microfluidic Platform for 3D Cultures of Mesenchymal Stem Cells, Towards Engineering Developmental Processes.. *Sci Rep.*, 18 May 2015, vol. 5, 10288
- **KWAPISZEWSKA K** ; **MICHALCZUK A** ; **RYBKA M** ; **KWAPISZEWSKI R** ; **BRZÓZKA Z.** A microfluidic-based platform for tumour spheroid culture, monitoring and drug screening.. *Lab Chip.*, 21 June 2014, vol. 14 (12), 2096-104
- **JIN, H.-J** ; **CHO, Y.-H** ; **GU, J.-M.** ; **KIM, J.** ; **OH, Y.-S.** A multicellular spheroid formation and extraction chip using removable cell trapping barriers.. *Lab Chip*, 2011, vol. 11, 115-119, http://dx.doi.org/10.1039/c01-c00134a
- **HSIAO, A. Y. et al.** Microfluidic system for formation of PC-3 prostate cancer co-culture spheroids.. *Biomaterials*, 2009, vol. 30, 3020-3027
- **TORISAWA, Y. et al.** A multicellular spheroid array to realize spheroid formation, culture, and viability assay on a chip.. *Biomaterials*, 2007, vol. 28, 559-566
- **WU, L. Y., DI CARLO, D. & LEE, L. P.** Microfluidic self-assembly of tumour spheroids for anticancer drug discovery. . *Biomed. Microdevices*, 2008, vol. 10, 197-202
- **MESSNER S** ; **AGARKOVA I** ; **MORITZ W** ; **KELM JM.** Multi-cell type human liver microtissues for hepatotoxicity testing.. *Arch Toxicol.*, 11 November 2012, vol. 87 (1), 209-13